# EUROPÄISCHE PATENTANMELDUNG

(11) **EP 0 994 182 A2**
(43) Veröffentlichungstag der Anmeldung: **19.04.2000**
(21) Anmeldenummer: 99116992.1
(22) Anmeldetag: 28.08.1999
(51) Int. Cl.: C12N 1/20

(54) **Verfahren zur Synchronisation von Zellteilungen mindestens einer Population in einer Kultur oder Mischkultur und zellzyklusabhängige Herstellung/Umsetzung von mindestens einer Substanz mit einer durch das Verfahren synchronisierten Population in einer Kultur oder Mischkultur**

(30) Priorität: 31.08.1998 DE 19839584
(71) Anmelder: Schulz, Christoph, Dr., 68199 Mannheim (DE)
(72) Erfinder: Schulz, Christoph, Dr., 68199 Mannheim (DE)

(57) **Zusammenfassung**

Das neue Synchronisationsverfahren soll die Zellphysiologie nicht beeinträchtigen, eine große Anzahl synchronisierter Zellen liefern und zellzyklusorientiert sein.

Die Erfindungsaufgabe wird gelöst, indem die Kulturmitglieder ein übertragenes/modifiziertes zellzyklusgekoppeltes Gen/Genprodukt besitzen und Substanzpulse in Zellzyklusabständen erfolgen, wobei die Substanz entweder wachstumhemmend oder eine Vorstufe einer wachstumsfördenden Substanz ist und durch das Gen/Genprodukt zellzyklusabhängig umgesetzt wird. Eine Zellsynchronisation resultiert, da nur Zellen wachsen können, die die Substanz inaktivieren bzw. zu der wachstumsfördenden Substanz umsetzen. Durch zusätzliche zellzyklusgekoppelte Gene/Genprodukte, die meßbare Substanzen erzeugen, können die Substanzpulsdauern/Pulsabstände regelkreisartig gesteuert werden. Eine zellzyklusabhängige Umsetzung/Herstellung von Substanzen wird durch weitere zellzyklusgekoppelte Gene/Genprodukte erreicht.

Das Synchronisationsverfahren kann zur Zellsynchronisation und zur zellzyklusabhängigen Herstellung/Umsetzung von Substanzen eingesetzt werden.

## Beschreibung

Die vorliegende Erfindung betrifft ein Verfahren zur Synchronisation von Zellteilungen mindestens einer Population in einer Kultur oder Mischkultur. Weiterhin sollen mit diesem Verfahren eine Gewinnung von zellzyklussynchronisierten Zellen, eine zellzyklusabhängige oder zellzyklusabhängig gemachte Herstellung von Substanzen, wie Proteinen, Enzymen, niedermolekularen Verbindungen, usw., und/oder eine zellzyklusabhängige Umsetzung von Substanzen erreicht werden.

Fur eine Vielzahl von Organismen, Mikroorganismen, Zellinien, Hybridomen usw. sind Verfahren zur Synchronisation von Zellteilungen einer Population bekannt.

Die bekannten Verfahren zur Synchronisation von Zellteilungen einer Population können grundsätzlich unterteilt werden in physikalische Verfahren, bei denen die Zellen der Population, die sich in einem bestimmten Abschnitt des Zellzyklus befinden, isoliert werden, und in physiologische Verfahren, bei denen die Zellen der Population in einem bestimmten Abschnitt des Zellzyklus angehäuft werden.

Zu den physikalischen Verfahren zählen die Percoll-Zentrifugation (z.B.: R D. Dwek, L. H. Kobrin, N. Grossman und E. Z. Ron. Synchronization of cell division in microorganisms by percoll gradients. J of Bacteriol, 1980, **144**: 17-21) und andere Gradientenzentrifugationstechniken (z.B.: J. M. Mitchison and W. S. Vincent. Preparation of synchronous cultures by sedimentation. Nature, 1965, **205**: 987-989; R. K. Poole. Fluctuations in buoyant density during the cell cycle of *Escherichia coli* K-12; significance for the preparation of synchronous cultures by age selection. J. Gen. Microbiol., 1977, **98**: 177-186; T. S. Sitz und R. R. Schmidt. Purification of *Synechococcus lividus* by equilibrium centrifiigation and its synchronization by differential centrifugation. J. Bacteriol, 1973, **115**: 43-46), das Sedimentationsverfahren (z.B.: T. Ooka und J. Daillie. A new method for cell culture synchronisation. Exp. Cell Res., 1974, **84**: 219-222), die Filtrationstechnik (z.B.: Y. Maruyama und T. Yanagita. Physical methods for obtaining synchronous culture of *Escherichia coli*. J. Bacteriol, 1956, **71**: 542-546), das Haften von Zellen an Membranen (z.B.: C. E. Helmstetter, Rate of DNA synthesis during the division cycle of *Escherichia coli* B/r. J. Mol. Biol., 1967, **24**: 417-427), Zellsortierung mittels Flußzytometrie (z.B.: P. N. Dean, J. W. Gray und Dolbeare. The analysis and interpretation of DNA distrubutions measured by cytometry. Cytometry, 1982, **3**: 188-195), das Abschütteln von mitotischen Zellen, auch 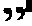 mitotic shake-off"- Verfahren oder mitotisches Selektions-Verfahren genannt (z.B.: T. Terasima und L. J. Tolmach. Growth and nucleic acid synthesis in synchronously dividing populations of HeLa cells. Exp. Cell Res., 1963, **30**: 344-362), die Begrenzung der Anwachszeit von trypsinierten Zellen (P. G. Held, J. W. Doylw, C. Sell und K. Janakidevi. Limited cell attachment time as a method to synchronize cells grown in monolayer culture. In Vitro Cell Dev. Biol., 1989, **25**: 1025-1030) und die Zentrifugalelutriationstechnik (z.B.: L. H. Johnston und A. L. Johnson. Elutriation of budding yeast. Methods Enzymol., 1997, **283**: 342-350). Vorteilhaft bei den physikalischen Verfahren ist die wesentlich schonendere Behandlung der Population im Vergleich zu den physiologischen Verfahren. Ein Nachteil der physikalischen Verfahren besteht darin, daß nur eine geringe Ausbeute an Zellen, die sich im gleichen Zellzyklusabschnitt befinden, und ein geringer Synchronisationsgrad erreicht wird. Ein weiterer Nachteil der physikalischen Verfahren besteht darin, daß bei Populationen, die Zellverbände oder die verklumpte Zellhaufen bilden, sich nur sehr schwer oder gar nicht Zellen, die sich in einen bestimmten Abschnitt des Zellzyklus befinden, isolieren lassen. Das 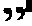 mitotic shake-off"-Verfahren und das Verfahren der Begrenzung der Anwachszeit von trypsinierten Zellen ist nur bei Populationen einsetzbar, die adhärent wachsen. Außerdem haben diese beiden Verfahren den Nachteil, arbeitsintensiv zu sein. Ebenfalls ist das Verfahren des Haftens von Zellen an Membranen nicht bei allen Populationen erfolgreich einsetzbar. Ein besonderer Nachteil besteht bei der Zellsortierung mittels Flußzytometrie und der Zentrifugalelutriationstechnik in den speziellen teuren Geräten, die benötigen werden und nicht zur normalen Laborausstattung gehören.

Die physiologischen Verfahren können unterteilt werden in nicht periodische Verfahren und periodische Verfahren. Zu den nicht periodischen Verfahren zählen die Blocktechniken, die Nährstoffbegrenzungsverfahren und die Synchronisation durch Temperaturschock (z.B.: B. Boucher und C. S. Norman. Cold synchronization for the study of peripheral blood and bone marrow chromosomes in leukemias and other hematologic disease states. Hum. Genet., 1980, **54**, 207-211). Bei den Blocktechniken werden eine Vielzahl von Substanzen eingesetzt (Eine Substanzliste findet sich im Gesamtkatalog 96/97 der Biomol Feinchemikalien GmbH, Hamburg, BRD, im Kapitel 9: Signal Transduction Unterkapitel Cell Cycle and Regulation. Eine aktuelle Liste ist auch im Internet unter http://www.biomol.com/12cycle.htm abrufbar.), die die Zellen der Population in einer bestimmten Phase des Zellzyklus reversibel blockieren und am weiteren Wachstum hindern, wie Methotrexat, Cycloheximid, Aphidicolin, Colcemid, Thymidin und Hydroxyharnstoff, nur um einige zu nennen. Außerdem lassen sich zu den Blocktechniken der Einsatz von 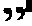 **C**ell-**D**ivision **C**ycle"-Mutanten als Mitglieder der zu synchronisierenden Population zählen (z.B.: L. L. Breeden. α-Factor synchronization of budding yeast. Methods Enzymol., 1997, **283**: 332-341), da diese Mitglieder der Population z.B. durch Temperaturerhöhung ebenfalls in einer bestimmten Phase des Zellzyklus reversibel blockiert werden. Zu den Nährstoffbegrenzungsverfahren gehören die Synchronisation durch Mangel an Isoleucin (z.B.: O. Garatun-Tjeldstø, I. F. Pryme, J. K. Weltman und R. M. Dowben. Synthesis and secretion of light-chain immunoglobulin in two successive cycles of synchronized plasmacytoma cells. J. Cell Biol., 1976, **68**, 232-239) oder an anderen Aminosäuren (z.B.: E. Z. Ron, S. Rozenhak und N. Grossman. Synchronization of cell division in *Escherichia coli* by amino acid starvation: stain specificity. J. Bacteriol., 1975, **123**, 374-376), durch Calciummangel (z.B.:T. Ashihara und R. Baserga. Cell synchronization. Methods Enzymol., 1979, **58**: 248-262), und durch Serumentzug (z.B.: K. Keyomarsi, L. Sandoval, V. Band und A. B. Pardee. Synchronization of tumor and normal cells from G₁ to multiple cell cycles by lovastatin. Cancer Res., 1991, **51**, 3602-3609), wobei beim Serumentzug vor allem benötigte Wachstumsfaktoren entzogen werden. Bei den periodischen Verfahren wird durch eine spezifische periodische Veränderung in den Kulturbedingungen eine Synchronisation der jeweiligen Population erreicht. Dazu werden in den bekannten Verfahren Temperaturverschiebungen, Änderungen der Lichtstärke (W. M. Darley und B. E. Volcani. Synchronized cultures: Diatoms. Methods Enzymol., 1971, **23**: 85-96), Änderungen bei einem organischen Nährstoff (z.B.: P. S. S. Dawson. Continuously synchronized growth. J. appl. Chem. Biotechnol., 1972, **22**, 79-103) oder anorganischen Nährstoff (z.B.: B. C. Goodwin. Synchronization of *Escherichia coli* in a chemostat by periodic phosphate feeding. European J. Biochem., 1969, **10**, 511-514) und periodische Verdünnungen der Kultur der jeweiligen Population (z.B.: R. G. Cutler und J. E. Evans. Synchronization of bacteria by a stationary-phase method. J. Bacteriol, 1966, **91**, 469-476) verwandt.

Vorteilhaft bei den physiologischen Verfahren gegenüber den physikalischen Verfahren ist, daß eine größere Ausbeute an synchronisierten Zellen erreicht und eine Population mit einer großen Zellzahl synchronisiert werden kann. Nachteilig bei den physiologischen Verfahren ist, daß in die Physiologie der Zellen der jeweiligen Zellenpopulation eingegriffen wird und so Synchronisationsartefakte induziert werden können. Besonders gilt das für die Substanzen bei den Blocktechniken, die zum Teil bei zu langer Einwirkungszeit toxisch für die jeweilige Population sind. Bei den Blocktechniken, die keine CDC-Mutanten einsetzen, ist auch bekannt, daß sich einige Populationen, wie z.B. von Tumorzellinien, nicht immer erfolgreich synchronisieren lassen. Ein besonderer Nachteil der nicht periodischen physiologischen Verfahren besteht bei der Synchronisation von Eukaryonten, bei denen die Teilung der nukleinsäurehaltigen Organellen, wie die der Mitochondrien (z.B.: S. B. Haase und D. J. Lew. Flow cytometric analysis of DNA content in budding yeast. Methods Enzymol., 1997, **283**: 322-332), nicht mitsynchronisiert wird. Die physikalischen und die nicht periodischen physiologischen Verfahren haben den großen Nachteil, daß die jeweilige Population nach einigen Zellzyklen beginnt asynchron zu wachsen. Ein weiterer Nachteil bei allen bekannten Verfahren stellen ruhende Zellen dar, die nicht mitsynchronisiert werden und jederzeit wieder beginnen können zu wachsen.

Um den Synchronisationsgrad und/oder die Ausbeute an synchronisierten Zellen zu erhöhen, wird oft eine Kombination der oben genannten bekannten Verfahren zusammen angewendet (z.B.: G. Cao, L.-M. Liu und S. F. Cleary. Modified method of mammalian cell synchronization improves yield and degree of synchronization. Exp. Cell Res. 1991, **193**, 405-410). Außer dem Nachteil des Mehraufwandes, um eine Population zu synchronisieren, ist es auch nachteilig, daß die eingesetzten Verfahren aufeinander abgestimmt werden müssen. Viele der Nachteile der einzelnen Verfahren bleiben auch bei einer Kombination miteinander bestehen oder verstärken sich sogar, wie zum Beispiel bei einer Kombination von verschiedenen Blocktechniken die Toxizität der eingesetzten Substanzen zusammen auf die Population wirkt.

Für eine Vielzahl von Stoffen ist bekannt, daß Ihre Konzentration in Zellen im Laufe der verschiedenen Zellzyklusphasen schwankt. Die bekannten Verfahren zur Synchronisation von Zellteilungen einer Population werden aufgrund der oben erwähnten Nachteile industriell bisher nur zur Gewinnung von zellzyklussynchronisierten Zellen zur Herstellung von Immundiagnostika eingesetzt. Für eine zellzyklusabhängige oder zellzyklusabhängig gemachte Herstellung von Substanzen, wie Proteinen, Enzymen, niedermolekularen Verbindungen usw., und/oder für eine zellzyklusabhängige Umsetzung von Substanzen sind die bekannten Verfahren zur Synchronisation von Zellteilungen einer Population aufgrund der obigen genannten Nachteile nur bedingt oder gar nicht einsetzbar.

Der Erfindung liegt die Aufgabe zugrunde, ein Verfahren zur Synchronisation von Zellteilungen einer Kultur oder Mischkultur mit jeweils mindestens zwei Mitgliedern mindestens einer Population zu haben, das die Physiologie der synchronisierten Mitglieder der Population kaum oder gar nicht beeinträchtigt, am Zellzyklus der synchronisierten Mitglieder der Population orientiert ist, eine andauernde Synchronisation ermöglicht, bei vielen verschiedenen Organismen, Mikroorganismen, usw. angewendet werden und eine große Anzahl von synchronisierten Zellen liefern kann.

Diese Aufgabe wird erfindungsgemäß dadurch gelöst, daß
a) die Mitglieder mindestens einer Population in der Kultur oder in der Mischkultur mindestens ein an mindestens ein zellzyklusabhängiges Gen oder Genprodukt direkt und/oder indirekt über Expressionsmechanismen und/oder andere zelluläre Regulationsmechanismen gekoppeltes eingebrachtes oder verändertes Gen oder Genprodukt besitzen, das durch diese Kopplung zellzyklusabhängig exprimiert oder aktiviert wird, und daß
b) mindestens eine Substanz, die entweder eine Vorstufe für mindestens eine mindestens wachstumsfördernde Substanz für die besagten Mitglieder der Population von a) ist, die durch das besagte gekoppelte Gen oder Genprodukt von a) direkt oder indirekt in die wachstumsfördernde Substanz umgesetzt wird, oder eine mindestens wachstumshemmende Substanz für die besagten Mitglieder der Population von a) ist, die durch das besagte gekoppelte Gen oder Genprodukt von a) direkt oder indirekt inaktiviert wird, oder eine Vorstufe für eine erst innerhalb der besagten Mitglieder der Population von a) zu einer mindestens wachstumshemmenden werdenden Substanz für die besagten Mitglieder der Population von a) ist, die und/oder dessen Folgeprodukt durch das besagte gekoppelte Gen oder Genprodukt von a) direkt oder indirekt inaktiviert wird, mindestens zweimal pulsartig und in zeitlichen Abständen gleich oder größer als die minimale Zeitdauer eines kompletten Zellzyklus/Teilungszyklus der besagten Mitglieder der Population von a) im jeweilig benutzten Kulturmedium verabreicht wird, so daß nur diejenigen besagten Mitglieder der Population von a) die zugeführte Substanz umsetzen/inaktivieren können, die innerhalb der Verabreichungszeit und in den Verabreichungsabständen das entsprechende gekoppelte Gen exprimieren oder über das entsprechende aktive Genprodukt verfügen.

Weitere Besonderheiten ergeben sich aus den Unteransprüchen.

Alle Organismen, Mikroorganismen, humane Zellinien, Zellinien von Säugetieren, Insektenzellinien, Pflanzenzellinien, Hybridome usw., die sich in einer Kultur oder Mischkultur kultivieren lassen, sind zur Synchronisation mit dem erfindungsgemäßen Verfahren geeignet, wenn erstens die unten beschriebene molekularbiologische Modifikation der Organismen, Mikroorganismen, usw. durchgeführt wurde, die je nach den gewünschten Anforderungen ohne erfinderisches Zutun im Rahmen der beanspruchten Lehre aus dem Stand der Technik herleitbar ist, und wenn zweitens eventuelle andere Organismen, Mikroorganismen, usw. in der Kultur oder Mischkultur, die für das Wachstum des zu synchronisierenden Organismen, Mikroorganismen, usw. nötig sind, nicht durch die pulsweise Zugabe der jeweils benutzten Substanz in ihrer Funktion beeinträchtigt werden. Der zweite Fall kann durch eine geeignete Wahl der jeweils benutzten Substanz, indem diese Organismen, Mikroorganismen, usw. nach entsprechender molekularbiologischer Modifikation ebenfalls mit der jeweils benutzten Substanz synchronisiert werden, oder durch Ersatz durch andere passende Organismen, Mikroorganismen, usw., die gleichermaßen das Wachstum des zu synchronisierenden Organismen, Mikroorganismen, usw. unterstützen, vermieden werden.

Zunächst werden die Kombinationen der pulsweise dem Medium zugesetzter Substanz, die mindestens wachstumshemmend für die jeweiligen Mitglieder der Population oder eine Vorstufe für eine erst innerhalb der jeweiligen Mitglieder der Population zu einer mindestens wachstumshemmenden werdenden Substanz ist, und des an ein zellzyklusabhängiges Gen/Genprodukt gekoppelten Gens/Genprodukts erläutert. Im Anschluß daran werden dann die Kombinationen der pulsweise dem Medium zugesetzter Vorstufe für eine Substanz, die mindestens wachstumsfördernd für die jeweiligen Mitglieder der Population ist, und des an ein zellzyklusabhängiges Gen/Genprodukt gekoppelten Gens/Genprodukts behandelt.

Als Kombinationen der pulsweise dem Medium zugesetzter Substanz, die mindestens wachstumshemmend für die jeweiligen Mitglieder der Population oder eine Vorstufe für eine erst innerhalb der jeweiligen Mitglieder der Population zu einer mindestens wachstumshemmenden werdenden Substanz ist, und des an ein zellzyklusabhängiges Gen/Genprodukt gekoppelten Gens/Genprodukts sind verschiedene Toxine geeignet, wie z.B. Antibiotika, Herbizide, Inzektizide, usw., und ihr zugehöriges Resistenzgen mit seinem Genprodukt, das die zugesetzte Substanz und/oder das wachstumshemmende Folgeprodukt durch eine Spaltung oder chemische Modifikation inaktiviert. Es sei ausdrücklich darauf hingewiesen, daß viele toxische Substanzen je nach vorliegender Konzentration im Medium und Verabreichungszeit unterschiedlich stark toxisch wirken oder auch nur wachstumshemmend sein können. Weiter sei ausdrücklich darauf hingewiesen, daß einige Substanzen erst durch Biotransformation in die eigentlichen toxischen Folgeprodukte umgesetzt werden (z.B.: das Insektizid Parathion durch eine Monooxgenase in das toxische Paraoxon, das Insektizid Demeton-S-methyl durch eine Monooxgenase in das toxische Demeton-S-methylsulfon, usw.) oder erst durch einen Synergisten die volle toxische Wirkung entfalten können.

Als Kombinationen von Antibiotika und zugehöriges Resistenzgen/Resistenzgenprodukt (z.B.: T. J. Foster. Plasmid-determined resistance to antimicrobial drugs and toxic metal ions in bacteria. Microbiol. Rev. 1983, **47**, 361-409) kommen z.B. in Frage:
- Penicillin G und diejenigen seiner Derivate (z.B.: Ampillicin, Carbenicillin, Oxacillin, Methicillin; Cloxacillin, usw.), die durch Spaltung von den verschiedensten Mikroorganismen stammenden Penicillinasen, Penicillin acyl esterasen, Penicillinamidasen inaktiviert werden, mit den dazugehörigen Penicillinasen, Penicillin acyl esterasen, Penicillinamidasen. Da von Pivampicillin bekannt ist, daß es erst durch Biotransformation in das toxische Ampicillin umgewandelt wird, kommt dieses Penicillinderivat ebenfalls in Frage.
- Cephalosporin C und diejenigen seiner Derivate (z.B.: Cephaloridin, usw.), die durch Spaltung von den verschiedensten Mikroorganismen stammenden Penicillinasen, Penicillin acyl esterasen, Penicillinamidasen inaktiviert werden, mit den dazugehörigen Penicillinasen, Penicillin acyl esterasen, Penicillinamidasen.
- Chloramphenicol mit den verschiedenen Typen von Chloramphenicol-acetyltransferasen aus den verschiedensten Mikroorganismen, die Chloramphenicol durch Acetylierung inaktivieren.
- diejenigen Mitglieder der Aminoglykos-Aminocyclitole-Antibiotikafamilie (z.B.: Neomycin B, Neomycin C, Paromomycin, Butirosin B, Lividomycin, Kanamycin A, Kanamycin B, Kanamycin C, Amikacin, Tobramycin, Gentamycin C1a, Gentamycin C1, Gentamicin C2, Sisomicin, Netilmicin, Streptomycin, usw.) mit den aus verschiedensten Mikroorganismen stammenden Aminoglykosid-O-Phosphotransferasen, die durch Phosphorylierung diese Mitglieder der Aminoglykoside-Aminocyclitole-Antibiotikafamilie inaktivieren.
- diejenigen Mitglieder der Aminoglykoside-Aminocyclitole-Antibiotikafamilie (z.B.: Neomycin B, Neomycin C, Paromomycin, Butirosin B, Lividomycin, Kanamycin A, Kanamycin B, Kanamycin C, Amikacin, Tobramycin, Gentamycin C1a, Gentamycin C1, Gentamicin C2, Sisomicin, Netilmicin, usw.) mit den aus verschiedensten Mikroorganismen stammenden Aminoglykosid-N-Acetyltransferasen, die durch Acetylierung diese Mitglieder der Aminoglykoside-Aminocyclitole-Antibiotikafamilie inaktivieren.
- diejenigen Mitglieder der Aminoglykoside-Aminocyclitole-Antibiotikafamilie (z.B.: Neomycin B, Neomycin C, Paromomycin, Butirosin B, Lividomycin, Kanamycin A, Kanamycin B, Kanamycin C, Amikacin, Tobramycin, Gentamycin C1a, Gentamycin C1, Gentamicin C2, Sisomicin, Netilmicin, Streptomycin, Spectinomycin, usw.) mit den aus verschiedensten Mikroorganismen stammenden Aminoglykosid-O-nukleotidyltransferasen, die durch Adenylierung diese Mitglieder der Aminoglykoside-Aminocyclitole-Antibiotikafamilie inaktivieren.

Als Kombinationen von Herbizid und zugehöriges Resistenzgen/Resistenzgenprodukt kommen z.B. in Frage:
- Phosphinothricin mit der aus verschiedensten Mikroorganismen stammenden Phosphinothricin-Acetyltransferase (z.B.: *Streptomyces viridochromogenes*), die Phosphinothricin durch Acetylierung inaktiviert.
- Bromoxynil mit der aus verschiedensten Mikroorganismen stammenden Bromoxynil-Nitrilase (z.B.: *Klebsiella ozaenae*), die die Cyano-Gruppe des Bromoxynil in eine Carboxy-Gruppe überführt.
- 2,4-Dichlorphenoxyessigsäure mit der aus verschiedensten Mikroorganismen stammenden 2,4-D-Monooxygenase (z.B.: *Alcaligenes eutrophus*).

Als Kombinationen der pulsweise dem Medium zugesetzter Vorstufe für eine Substanz, die mindestens wachstumsfördernd für die jeweiligen Mitglieder der Population ist, und des an ein zellzyklusabhängiges Gen/Genprodukt gekoppelten Gens/Genprodukts sind verschiedene Vorstufen für essentielle Substanzen geeignet, wie z.B. für Aminosäuren, Vitamine, usw., und eines der zugehörigen Gene mit seinem Genprodukt, das die Vorstufe in die wachstumsfördende Substanz überführt. Es sei ausdrücklich darauf hingewiesen, daß einige wachstumsfördende Substanzen bei einigen Organismen, wie die Aminosäure Histidin, zu bestimmten Wachstumszeiten essentiell sind und zu anderen Zeiten das Wachstum lediglich fördern.

Als Kombinationen von Vorstufe bei der Biosynthese der essentiellen Aminosäure und zugehöriges Gen/Genprodukt, um die Vorstufe in essentielle Aminosäure zu überführen, kommen z.B. in Frage:
- α-Keto-β-methylvalerat (Vorstufe für Isoleucin) oder α-Ketoisovalerat (Vorstufe für Valin) mit der aus verschiedensten Mikroorganismen stammenden Valin-Aminotransferase, die die Vorstufen in die essentiellen Aminosäuren umsetzt.
- α-Ketoisocaproat (Vorstufe für Leucin) mit der aus verschiedensten Mikroorganismen stammenden Leucin-Aminotransferase, die die Vorstufe in die essentielle Aminosäure umsetzt.
- Saccharopin (Vorstufe für Lysin bei Pilzen) mit der Saccharopin-Dehydrogenase, die die Vorstufe in die essentielle Aminosäure umsetzt.
- Homoserinphosphat (Vorstufe für Threonin bei Bakterien) mit der Threonin-Synthase, die die Vorstufe in die essentielle Aminosäure umsetzt.
- Homocystein (Vorstufe für Methionin bei Bakterien) mit der Betain-Homocystein-Methyltransferase oder der Dimethylthetin-Homocystein-Methyltransferase, die die Vorstufe in die essentielle Aminosäure umsetzen. Bei dieser Kombination ist zu beachten, daß die Mitglieder keine funktionelle 5-Methyltetrahydrofolat:Homocysteinmethyltransferase (ein Enzym für die Wiederverwertung) besitzen dürfen und eventuell Betain bzw. Dimethylthetin dem jeweiligen Medium zugesetzt werden muß.

Als Kombinationen von Vorstufe bei der Biosynthese des essentiellen Vitamins und zugehöriges Gen/Genprodukt, um die Vorstufe in das essentielle Vitamin zu überführen, kommen z.B. in Frage:
- Pantoat und β-Alanin (Vorstufen für Pantothenat bei Bakterien) mit der Pantoat -β-Alanin-Ligase, die die Vorstufen in das Vitamin umsetzt. Es ist zweckmäßig bei dieser Kombination, daß β-Alanin pulsweise dem Medium mit beigesetzten Pantoat zugeführt wird.
- L-Gulonolacton (Vorstufe für Ascorbat) mit der L-Gulonolacton-Oxidase, die die Vorstufe in das Vitamin umsetzt.

Der Fachmann für einen Organismus, Mikroorganismus, usw. kennt die genauen Bedingungen und Bedürfnisse zur Kultivierung des Organismus, Mikroorganismus, usw. Der Fachmann kann deshalb aufgrund seines Fachwissens über den zu synchronisierenden Organismus, Mikroorganismus, usw. ohne erfinderisches Zutun die geeignete Kombination aus den oben nicht in einschränkender Weise aufgeführten Kombinationen je nach den gewünschten Anforderungen auswählen. Zum Beispiel bietet sich für einen Fachmann für humane Zellinien als Kombination die Vorstufe für eine essentielle Aminosäure und ein entsprechendes Gen für ein Enzym an, das die Vorstufe in die entsprechende Aminosäure überführt. Bei der Durchführung des erfindungsgemäßen Verfahrens werden bis auf die Anpassung zur Pulserzeugung die üblichen Kulturbedingungen und Kulturgefäße für den jeweiligen Organismus, Mikroorganismus, usw. eingehalten. Der Fachmann kann gegebenenfalls den Organismus, Mikroorganismus, usw. je nach Anforderungen gegen eine Substanz sensitiv oder bedürftig machen mit den bekannten Mutationsverfahren für den Organismus, Mikroorganismus, usw. ohne erfinderisches Zutun im Rahmen der beanspruchten Lehre aus dem Stand der Technik.

Als zellzyklusabhängiges Gen oder Genprodukt der Mitglieder der Population, an das über direkte und/oder indirekte Expressionsmechanismen und/oder andere zelluläre Regulationsmechanismen das eingebrachte oder veränderte Gen oder Genprodukt gekoppelt sein soll, sind besonders diejenigen Cycline geeignet, deren Zellkonzentration im Verlauf des Zellzyklus regelmäßige Schwankungen aufweist, wie z.B. die G1-Cycline Cln1 und Cln2. Die Zellkonzentration dieser Cycline, die wiederum mit anderen Zellzyklusproteinen interagieren, wird in komplexer Weise einerseits durch die Expression an der jeweiligen mRNA und anderseits des Abbaus des jeweiligen Cyclins über den Ubiquitinweg reguliert. Alternativ sind statt der Cycline auch andere bekannte zellzyklusabhängige Proteine/Enzyme, deren Zellkonzentration im Verlauf des Zellzyklus regelmäßige Schwankungen aufweist, geeignet.

Als Kopplungsart kommt zum Beispiel eine Fusion des übertragenen Gens mit einem dieser bekannten Cyclingene derjeweiligen Mitglieder der Population in Frage. Als Position der Fusion des übertragenen Gens mit dem jeweiligen Cyclingen ist besonders die Region des Cyclingens geeignet, die die Region des Proteins darstellt, die nach der sogenannten PEST-Region im C-terminalen Ende des jeweiligen Cyclinproteins liegt. Alle anderen Bereiche der Cycline sind nicht oder nur bedingt geeignet, da diese entweder für die Steuerung des Abbaus dienen (vor allem die PEST-Region und die Destruction Box) oder für die Wechselwirkung mit anderen Zellzyklusproteinen wichtig sind (vor allem die Cyclin Box). Durch diese Art der Kopplung wird das übertragene Gen und sein Genprodukt gleichfalls zellzyklusabhängig exprimiert und abgebaut. Ein eigener Promoter und Transkriptionsterminationssequenzen erübrigen sich bei dieser Art von Kopplung. Bei Bakterien, Hefen und vielen anderen Mikroorganismen ist bekannt, daß durch homologe Rekombination das übertragene Genkonstrukt stabil in das Genom integriert wird. Ein geeignetes Genkonstrukt für die Transformation/Transfektion (z.B. mittels Elektroporation) besteht aus einem der für diese Mikroorganismen oder Organismen jeweilig bekannten Vektoren, der das Gen, flankiert mit dem oben genannten Bereich des gewünschten Cyclins, trägt. Ein gemeinsames Leseraster vom übertragenen Gen und der flankierenden Bereiche mit der Cyclingensequenz muß bei der Herstellung des Genkonstrukts gewährleistet sein, damit später ein funktionelles Proteinfusionsprodukt resultiert. Bei anderen Organismen, wie Pflanzen, humanen Zellinien, Insektenzellinien, usw. ist bekannt, daß bei der direkten Transformation (z.B.: J. Paszkowski, R.D. Shillito, M. Saul, V. Mandák, T. Hohn, B. Hohn und J. Potrykus. Direct gene transfer to plants. EMBO J., 1984, **3**, 2717-2722) von beliebigen DNA-Stücken eine stabile zufällige Integration ins Genom stattfindet, also auch in dem oben erwähnten Bereich der Cyclingene und in anderen zellzyklusabhängigen Genen. Als ein geeignetes Genkonstrukt, um diese Transformationsart durchzuführen, kommt bei diesen Organismen in Frage z.B. ein Produkt der Polymerasekettenreaktion des kompletten Gens ohne Introns, wenn auf die Introne verzichtet werden kann, oder im anderen Fall ein DNA-Restriktionsfragment, welches das komplette Gen mit Introns enthält. Falls ein zusätzlich übertragenes Cyclingen zu keinen Problemen im Organismus, Mikroorganismus, usw. führt, kann als geeignetes Genkonstrukt alternativ ein passender bekannter Vektor für den jeweiligen Organismus, Mikroorganismus, usw. eingesetzt werden, welcher erstens ein passendes Cyclingen mit einem passenden Promotor, zweitens das zu übertragende Gen, das im oben genannten Bereich des Cyclinsgens integriert ist, und drittens Transkriptionsterminationssequenzen, die der jeweilige Organismus, Mikroorganismus, usw. erkennt, besitzt. Besonders geeignet ist ein passendes Cyclingen vom jeweiligen Organismus, Mikroorganismus, usw. mit seinem Promotor. Ein gemeinsames Leseraster des Genkonstrukts muß bei der Herstellung des Genkonstrukts gewährleistet sein, damit später ein funktionelles Proteinfusionsprodukt resultiert.

Die Herstellung der oben genannten Genkonstrukte und die Durchführung der Transformation/Transfektion des jeweiligen hergestellten Genkonstrukts ist je nach den gewünschten Anforderungen ohne erfinderisches Zutun im Rahmen der beanspruchten Lehre aus dem Stand der Technik herleitbar. Ebenso kann die Transformations/Transfektionseffizienz und die Anzahl an zu erwartenden geeigneten Transformaten/Transfektanten ohne erfinderisches Zutun aus dem Stand der Technik des jeweilig eingesetzten Transformations/Transfektionsverfahrens abgeleitet werden.

Mit den bekannten Verfahren zur Selektion und Anreicherung können die benötigten molekularbiologisch veränderten Mitglieder der Population für das erfindungsgemäße Verfahren nach der erfolgten Transformation/Transfektion nur sehr bedingt oder gar nicht angereichert und selektiert werden, da bei diesen Verfahren Selektionsbedingungen vorliegen, die Transformanten/Transfektanten mit einer nicht zellzyklusabhängigen induktiven/konstitutiven Expression des übertragenen Gens beim Wachstum begünstigen. Da bei dem erfindungsgemäßen Verfahren durch die pulsweise Substanzzugabe und dem Zeitabstand der Substanz-Pulse von der Dauer mindestens eines Zellzyklus Selektionsbedingungen vorliegen, die Transformanten/Transfektanten mit einer zellzyklusabhängigen Expression des übertragenen Gens beim Wachstum begünstigen, wird vorzugsweise das erfindungsgemäße Verfahren zur Selektion und Anreicherung der benötigten molekularbiologisch veränderten Mitglieder der Population für das erfindungsgemäße Verfahren eingesetzt. Bei Organismen, Mikroorganismen, usw., die auf festen Medien wachsen, kann das erfindungsgemäße Verfahren direkt als Screening-Verfahren zur Ermittlung geeigneter molekularbiologisch modifizierter Mitglieder der Population eingesetzt werden. Die Transformanten/Transfektanten müssen bei den anderen Organismen, Mikroorganismen, usw. zunächst separiert werden, bevor das erfindungsgemäße Verfahren eingesetzt werden kann.

Die weitere Eingrenzung auf besonders günstige molekularbiologisch modifizierte Mitglieder der Population und zur Charakterisierung der einzelnen Transformaten/Transfektanten ist je nach den gewünschten Anforderungen ohne erfinderisches Zutun im Rahmen der beanspruchten Lehre aus dem Stand der Technik herleitbar. Beispielsweise kann die Polymerasekettenreaktion zur Detektion des in das Genom integrierten Fusionsprodukts und/oder die Durchflußzytometrie zur Bestimmung der Expressionsstärke des übertragenen Gens im Verlauf des Zellzyklus dienen.

Der Puls der verabreichten Substanz im Medium kann auf mehrere Arten erfolgen.

Für den Fall, daß die Mitglieder der Population adhärent wachsen oder an den Wänden des Kulturgefäßes fest haften, kann ein Puls von beliebiger Größe und Dauer durch Austausch von Medium ohne Substanz und Medium mit einer geeigneten Konzentration von Substanz erzeugt werden, wobei der Austausch vorsichtig zu geschehen hat, damit die Zellen nicht vom Kulturgefäß abgelöst werden.

Bei Suspensionskulturen kann der Substanz-Puls dadurch erzeugt werden, indem erstens dem Medium ein die Substanz umsetzendes Enzym in freier oder an einem Träger gebundener Form und eventuell benötigte zusätzliche Enzymsubstrate in geeigneter Konzentration zugesetzt wird und zweitens eine bestimmte Menge an Substanz dem Medium auf einmal zugeführt wird. Die Substanz wird dann innerhalb einer gewissen Zeitspanne umgesetzt bis eine sehr geringe nicht störende Restkonzentration und ein Substanz-Puls im Medium resultiert. Die Bindung des Enzyms an einen passenden Träger ist je nach den gewünschten Anforderungen ohne erfinderisches Zutun im Rahmen der beanspruchten Lehre aus dem Stand der Technik herleitbar.

Bei einer dem Medium zugesetzten Substanz, die mindestens wachstumshemmend für die jeweiligen Mitglieder der Population oder eine Vorstufe für eine erst innerhalb der jeweiligen Mitglieder der Population zu einer mindestens wachstumshemmenden werdenden Substanz ist, kommen besonders die Genprodukte des jeweils übertragenen Gens in Frage, um dem Medium zugesetzt zu werden mit ihren eventuellen noch benötigten Substraten.

Bei einer dem Medium, das die essentielle Aminosäure nicht enthalten darf, zugesetzten Vorstufe für eine essentielle Aminosäure kommen z.B. in Frage:
- bei α-Keto-β-methylvalerat (Vorstufe für Isoleucin), α-Ketoisovalerat (Vorstufe für Valin) oder α-Ketoisocaproat (Vorstufe für Leucin) das Enzym α-Ketoisovalerat-Dehydrogenase und eventuelle benötigte zusätzliche Substrate, die das Enzym für die Umsetzung benötigt.
- bei Saccharopin (Vorstufe für Lysin bei Pilzen) das Enzym Saccharopin-Dehydrogenase und eventuelle benötigte zusätzliche Substrate, die das Enzym für die Umsetzung benötigt.
- bei Homoserinphosphat (Vorstufe für Threonin bei Bakterien) die Enzyme Cystathionin-β-synthase und Cystathionin-γ-synthase aus Pflanzen und eventuelle benötigte zusätzliche Substrate, die die Enzyme für die Umsetzung benötigen.
- bei Homocystein (Vorstufe für Methionin bei Bakterien) das Enzym Cystathionin-γ-lyase und eventuelle benötigte zusätzliche Substrate, die das Enzym für die Umsetzung benötigt.

Bei einer dem Medium, das das Vitamin nicht enthalten darf, zugesetzten Vorstufe für ein Vitamin kommen z.B. in Frage:
- bei β-Alanin (eine Vorstufe für Pantothenat bei Bakterien) das Enzym 4-Aminobutyrattransaminase und eventuelle benötigte zusätzliche Substrate, die das Enzym für die Umsetzungbenötigt.

Die Pulsdauer und Pulsform bei den verschiedenen zugesetzten Substanzen hängen von der dem Medium zugesetzten Menge an Enzym ab, dem üblicherweise verwendeten Medium, den verwendeten Kulturgefäßen und den Kulturbedingungen, und muß deshalb empirisch eingestellt werden. Zur Variation der Pulsdauer kann man erstens unterschiedliche Substanz-Konzentrationen einsetzen und die Menge des dem Medium zugesetztem Enzyms konstant halten oder zweitens eine bestimmte Substanz-Konzentration verwenden und unterschiedliche Mengen des dem Medium zugesetztem Enzyms einsetzen. Alternativ kann, wenn aus verschiedenen Gründen kein Enzym dem Medium zugesetzt werden kann, auch an einen Träger gebundener spezifischer Ligand für die zugesetzte Substanz dem Medium zugegeben werden, der die zugesetzte Substanz durch spezifische Bindung aus dem Medium entfernt. Die Pulsdauer und Pulsform bei den verschiedenen zugesetzten Substanzen hängt dann von der dem Medium zugesetzten Menge der an dem Träger gebundenen Liganden ab, dem üblicherweise verwendeten Medium, den verwendeten Kulturgefäßen und den Kulturbedingungen, und muß ebenfalls empirisch eingestellt werden. Bei kontinuierlichen Kulturen oder Mischkulturen mit den molekularbiologisch modifizierten Mitgliedern der Population mit einer Zelldichte im Medium, die um einen konstanten Wert schwankt, kann die Substanz-Pulserzeugung des erfindungsgemäßen Verfahrens, nachdem die Kultur oder Mischkultur synchronisiert und die entsprechende Zelldichte erreicht ist, nach Prüfung auf Eignung umgestellt werden. Dies ist der Fall, wenn durch die synchronisierten Mitglieder der Population die zugesetzte Substanz auf eine sehr geringe Restkonzentration im Medium ohne zugesetztes Enzym bzw. zugesetzten spezifischen Ligand innerhalb der ursprünglichen Pulsdauer umgesetzt/inaktiviert wird.

Eine andere Art der Pulserzeugung kann durch spezielle Kulturgefäße erreicht werden, bei denen Substanzen sehr schnell aus dem Medium durch Dialyse/Filtrationsvorrichtungen, wie z.B. bei der Hohlfaserkultivierung, entfernt werden.

Falls die Generationszeit der Mitglieder der Population ausreichend lang ist, kann der Substanz-Puls erzeugt werden, indem die Kultur mit den Mitglieder der Population nach einer bestimmten Zeit der Substanzzugabe, schnell durch eine passende Säule mit einem Träger, die eines der oben genannten Enzyme und/oder einen spezifischen Liganden für die Substanz gebunden hat, mit einer passender Vorrichtung geführt wird. Die Substanz wird dabei umgesetzt/inaktiviert und/oder gebunden und ein Substanz-Puls resultiert. Verbleibende Mitglieder der Population müssen vor einer neuerlichen Passage über die Säule entfernt und/oder abgetötet werden.

Die Pulsdauern und die Zeitabstände zwischen den Substanz-Pulsen bei den jeweiligen molekularbiologisch veränderten Mitgliedern der Population in Kultur oder Mischkultur muß experimentell bestimmt werden. Dies kann am sinnvollsten bei der Anreicherung, Selektion und Screening der für das erfindungsgemäße Verfahren benötigten molekularbiologisch veränderten Mitglieder der Population geschehen. Die minimalsten Pulsdauern bei unterschiedlichsten durchschnittlichen Substanz-Konzentrationen im Substanz-Puls und die übliche Generationszeit für das jeweilige Medium, den Kulturgefäßen und Kulturbedingungen müssen zunächst ermittelt werden. Die minimalsten Pulsdauern bei den mindestens wachstumshemmenden Substanzen oder bei den Substanzen, die Vorstufen für eine mindestens wachstumshemmendes Folgeprodukt sind, werden bestimmt, indem Substanz-Pulse mit unterschiedlichen Zeitdauern und verschiedenen durchschnittlichen Substanz-Konzentrationen im Substanz-Puls den ursprünglichen Mitgliedern der Populationen in Kultur oder Mischkultur verabreicht werden.

Die minimalsten Pulsdauern bei den mindestens wachstumshemmenden Substanzen oder bei den Substanzen, die Vorstufen für eine mindestens wachstumshemmendes Folgeprodukt sind, sind diejenigen, die die ursprünglichen Mitglieder der Populationen am Wachstum hindern oder sogar abtöten. Bei den Vorstufen für eine mindestens wachstumsfördende Substanz können die minimalsten Pulsdauern nicht direkt bestimmt werden. Indem statt der Vorstufe für eine mindestens wachstumsfördende Substanz die wachstumsfördende Substanz mit unterschiedlichen Zeitdauern und verschiedenen Konzentrationen den ursprünglichen Mitgliedern der Populationen in Kultur oder Mischkultur verabreicht werden, können Richtwerte für die minimalsten Pulsdauern abgeschätzt werden. Die Richtwerte für die minimalsten Pulsdauern für die Vorstufe für eine mindestens wachstumsfördende Substanz sind diejenigen, die die ursprünglichen Mitgliedern der Populationen gerade zum Wachstum verhelfen. Die Zeitabstände zwischen den Substanz-Pulsen ergibt sich aus der Bestimmung der Generationszeit der jeweiligen ursprünglichen Mitglieder der Population im jeweiligen Medium und Kulturgefäßen. Die Zeitabstände der Substanz-Pulse sollten dann im erfindungsgemäßen Verfahren mit mindestens der Dauer der Generationszeit verabreicht werden, wobei zu beachten ist, daß durch die molekularbiologische Modifikation der ursprünglichen Mitglieder der Population je nach Modifikationsart eine Verlängerung der ursprünglichen Generationszeit möglich ist. Das erfindungsgemäße Verfahren wird mit einer Reihe von Medien, die unterschiedliche Pulsdauer und unterschiedliche Substanz-Konzentrationen im Puls besitzen, mit jeweils einer ausreichenden Menge an Transformanten/Transfektanten durchgeführt. Die Pulsdauern liegen bei der Reihe von Medien zwischen den minimalsten Pulsdauern und der zu bestimmenden Generationszeit. Nachdem eine ausreichende Anzahl an Pulsen, die ohne erfinderisches Zutun im Rahmen der beanspruchten Lehre aus dem Stand der Technik herleitbar ist, durchgeführt wurde oder eine Synchronisation der Kultur festgestellt werden kann und sich die benötigten Mitglieder der Population angereichert haben, werden die einzelnen Transformanten/Transfektanten separiert. Die einzelnen Transformanten/Transfektanten werden nach besonders geeigneten molekularbiologisch veränderten Mitgliedern der Population gescreent. Geeignete molekularbiologisch veränderte Mitglieder der Population besitzen eine Pulsdauer mit geringen durchschnittlichen Substanz-Konzentrationen im Puls nahe der minimalen Pulsdauer und wesentliche kürzere Pulsdauer als die Dauer der ursprünglichen Generationszeit, da bei diesen vor allem ein geeigneter Synchronisationsgrad zu erwarten ist. Die ermittelten Pulsdauern der jeweiligen molekularbiologisch veränderten Mitglieder der Population für das jeweilige Medium, den Kulturgefäßen und Kulturbedingungen dienen als Richtwert für die Anpassung des erfindungsgemäßen Verfahrens auf größere Kulturvolumina, andere Kulturbedingungen und andere Kulturgefäße. Die Abstände der Pulse kann nach Prüfung auf Eignung auf Vielfache oder Kombinationen von Vielfachen der ermittelten Generationszeit verändert werden, um geeignetere molekularbiologische Mitglieder der Population zu erhalten oder um die Population zu schonen, wenn mindestens wachstumshemmende Substanzen oder Vorstufen für mindestens wachtumshemmde Folgeprodukte eingesetzt werden.

Das erfindungsgemäße Verfahren kann so ausgestaltet werden, daß mehrere Organismen, Mikroorganismen, usw. in einer Kultur oder Mischkultur einzeln voneinander oder gemeinsam synchronisiert werden. Dies wird erreicht durch eine jeweils passenden Auswahl der oben aufgeführten Kombinationen von Substanzen und Genen/Genprodukten durch den Fachmann, und nachdem die jeweiligen Organismen, Mikroorganismen, usw. entsprechend molekularbiologisch modifiziert wurden. Eine weitere Ausgestaltung des erfindungsgemäße Verfahren ist, daß ein Organismus, Mikroorganismus, usw. in einer Kultur oder Mischkultur durch mehrere Substanzen, die entweder gleichzeitig oder in bestimmten Zeitabständen zueinander verabreicht werden, synchronisiert wird. Dies wird ebenfalls erreicht durch eine passenden Auswahl der oben aufgeführten Kombinationen von Substanzen und Genen/Genprodukten durch den Fachmann, und nachdem der jeweilige Organismus, Mikroorganismus, usw. entsprechend molekularbiologisch modifiziert wurde. Beide Ausgestaltungen des erfindungsgemäßen Verfahrens zu kombinieren ist ebenfalls möglich durch eine jeweils passende Auswahl der oben aufgeführten Kombinationen von Substanzen und Genen/Genprodukten durch den Fachmann, und nachdem die jeweiligen Organismen, Mikroorganismen, usw. entsprechend molekularbiologisch modifiziert wurden.

Sinnvoll wäre eine Steuerung der Pulsdauern und der Zeitabstände zwischen den Substanz-Pulsen, um eine optimale Synchronisation für die einzelnen Populationen in der Kultur oder Mischkultur während der ganzen Kultivierungszeit zu erhalten, insbesondere wenn sich gewisse Kulturbedingungen ändern, oder um eine bessere Anpassung an andere Kulturbedingungen, Kulturvolumina und/oder Kulturgefäßen zu erreichen.

Dies wird erreicht, indem die Substanzverabreichung beim Verfahren nach Patentanspruch 1, dadurch gekennzeichnet ist, daß mindestens ein weiteres zusätzlich eingebrachtes oder verändertes zellzyklusgekoppeltes Gen oder Genprodukt der Mitglieder der Population direkt oder indirekt mindestens ein Photon und/oder mindestens eine meßbare Substanz zellzyklusabhängig erzeugt, und daß die pulsartige Zugabe der besagten Substanz nach Patentanspruch 1 für die Mitglieder der Population auf das Auftretens des Photons und/oder auf das Auftreten der meßbaren Substanz abgestimmt wird.

Als weitere zusätzlich zellzyklusgekoppelte Gene oder Genprodukte der jeweiligen molekularbiologisch modifizierten Mitglieder der Population, die direkt oder indirekt mindestens ein Photon und/oder mindestens eine meßbare Substanz zellzyklusabhängig erzeugen, kommen z.B. in Frage:
- die Firefly Luciferase, verschiedene bakterielle Luciferasen, β-Galaktosidase, β-Glucuronidase (z.B.: I. Bronstein, J. Fortin, P. E. Stanley, G. S. A. B. Stewart und L. J. Kricka. Chemiluminescent and bioluminescent reporter gene assays. Anal. Biochem, 1994, **219**, 169-181).
- Das 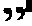 Green fluorescent protein" (GFP) von der Qualle *Aequorea victoria*, das blaues Licht absorbiert und grünes Licht emittiert (z.B.: K. Nabeshima, S. Saitoh und M. Yanagida. Use of green fluorescent protein for intracellular protein localization in living fission yeast cells. Methods Enzymol., 1997, **283**: 459-471).

Als Positionen in den Genkonstrukten, die bei den Transformation/Transfektionsverfahren, die die homologe Rekombination ausnutzen, eingesetzt werden, sind diejenigen besonders geeignet, die zwischen dem für die Synchronisation benötigten zusätzlichen Gen und einem der flankierenden Cyclinsequenzen liegen. Bei den Genkonstrukten, die das zur Synchronisation benötigte zusätzliche Gen und flankierende Cyclinsequenzen aufweisen, sind ebenfalls diejenigen Positionen besonders geeignet, die zwischen dem für die Synchronisation benötigten zusätzlichen Gen und einem der flankierenden Cyclinsequenzen liegen. Bei den anderen Genkonstrukten sind die flankierenden Positionen direkt am übertragenen Gen, das zur Synchronisation benötigt wird, geeignet. Ein gemeinsames Leseraster der verwendeten Gene muß bei der Herstellung der erweiterten Genkonstrukte gewährleistet sein, damit später ein funktionelles Fusionsprodukt resultiert. Es kann auch vorteilhaft sein, das zusätzliche Gen/Genprodukt mit einem anderen geeigneten Cyclingen des Organismus, Mikroorganismus, usw. zu koppeln als das übertragene Gen/Genprodukt, das für die Synchronisation benötigt wird, da dann das zusätzliche Gen/Genprodukt zu einem anderen Zeitpunkt im Zellzyklus am stärksten exprimiert wird als das übertragene Gen/Genprodukt für die Synchronisation.

Indem in zeitlicher Folge die Aktivität der zusätzlichen Genprodukte bestimmt wird, kann durch leichte Variation der Pulsdauer und Zeitabstände jeweils die optimalste Synchronisation für die einzelnen Populationen eingestellt werden. Die Bestimmung kann je nach Genprodukt und den eingesetzten Substraten für das Genprodukt auch in vivo (z.B.: F. F. Craig, A. C. Simmonds, D. Watmore, F. McCapra und M. R. H. White. Membrane-permeable luciferin esters for assay of firefly luciferase in live intact cells. Biochem J., 1991, **276**, 637-641) stattfinden, wobei durch die Umsetzung des jeweilig eingesetztes Substrats entweder ein Farbstoff gebildet oder Licht emittiert wird. Der Fachmann kann die Bestimmung der Aktivitäten der Genprodukte ohne erfinderisches Zutun im Rahmen der beanspruchten Lehre aus dem Stand der Technik herleiten.

Die molekularbiologisch modifizierten Mitglieder der Population produzieren zwar von sich aus bestimmte Substanzen zellzyklusabhängig und/oder setzen bestimmte Substanzen zellzyklusabhängig um Sinnvoll wäre aber noch eine gezielte zellzyklusabhängige Herstellung/Umsetzung von Substanzen. Die Herstellung oder Umsetzung mindestens einer Substanz mit mindestens einer mit dem Verfahren nach dem Patentanspruch 1 oder nach Patentanspruch 2 synchronisierten Kultur oder Mischkultur mit jeweils mindestens zwei Mitgliedern mindestens einer Population ist dadurch gekennzeichnet, daß die Mitglieder der Population mindestens ein weiteres zusätzlich eingebrachtes oder verändertes zellzyklusgekoppeltes Gen oder Genprodukt besitzen, das mindestens eine Substanz in einem bestimmten Zellzyklusabschnitt herstellt oder umsetzt.

Als weitere zusätzlich zellzyklusgekoppelte Gene/Genprodukte, die mindestens eine Substanz in einem bestimmten Zellzyklusabschnitt herstellen oder umsetzten, sind besonders passende Enzym- oder Proteingene geeignet, die in Verbindung mit den anderen übertragenen Genprodukten ein funktionelles Enzym oder Protein in dem jeweiligen Organismus, Mikroorganismus, usw. ergeben.

Die jeweiligen Enzym- oder Proteingene werden je nach Anforderungen mit Spaltungsstellen für Proteasen versehen, wenn diese von den anderen Genprodukten abgespalten werden sollen, und für die Transformation/Transfektion z.B. in die oben genannten Genkonstrukte integriert. Als Positionen in den Genkonstrukten, die bei den Transformation/Transfektionsverfahren, die die homologe Rekombination ausnutzen, eingesetzt werden, sind diejenigen besonders geeignet, die zwischen dem für die Synchronisation benötigten zusätzlichen Gen und einem der flankierenden Cyclinsequenzen liegen. Bei den Genkonstrukten, die das zur Synchronisation benötigte zusätzlichen Gen und flankierende Cyclinsequenzen aufweisen, sind ebenfalls diejenigen Positionen besonders geeignet, die zwischen dem für die Synchronisation benötigten zusätzlichen Gen und einem der flankierenden Cyclinsequenzen liegen. Bei den anderen Genkonstrukten sind die flankierenden Positionen direkt am übertragenen Gen, das zur Synchronisation benötigt wird, geeignet. Ein gemeinsames Leseraster der verwendeten Gene muß bei der Herstellung der erweiterten Genkonstrukte gewährleistet sein, damit später ein funktionelles Fusionsprodukt resultiert. Es kann auch vorteilhaft sein, das zusätzliche Gen/Genprodukt mit einem anderen geeigneten Cyclingen des Organismus, Mikroorganismus, usw. zu koppeln als das übertragene Gen/Genprodukt, das für die Synchronisation benötigt wird, da dann das zusätzliche Gen/Genprodukt zu einem anderen Zeitpunkt im Zellzyklus am stärksten exprimiert wird als das übertragene Gen/Genprodukt für die Synchronisation.

Nachdem die Mitglieder der Population mit dem erfindungsgemäßen Verfahren synchronisiert wurden, kann im Fall einer Herstellung einer Substanz die Kultivierung zu einem günstigen Zeitpunkt abgebrochen werden. Zum Beispiel ist ein günstiger Zeitpunkt, wenn das zusätzlich übertragene Gen/Genprodukt am stärksten die Substanz exprimiert oder störende andere Substanzen die geringste Konzentration innerhalb der Mitglieder und/oder im Medium aufweisen. Der Fachmann kann die Isolation der jeweils hergestellten Substanz ohne erfinderisches Zutun im Rahmen der beanspruchten Lehre aus dem Stand der Technik herleiten.

Im anderen Fall, wenn eine Substanz umgesetzt werden soll, kann es z.B. günstig sein, die Substanz nur zu gewissen Zeitpunkten des Zellzyklus umzusetzen, da zu diesen Zeitpunkten eine bessere Umsetzung stattfindet oder weniger störende andere Substanzen entstehen. Dies ist besonders wichtig für die hochreine Herstellung/Umsetzung von Substanzen.

Es sei ausdrücklich darauf hingewiesen, daß für das Verfahren benötigte DNA-Sequenzen, wie z.B. für Cyclingene, bei den üblichen Genbanken (wie EMBL, GenBank, usw.) in sehr großer Anzahl vorhanden sind. Gleichfalls sind bei den üblichen Stammsammlungen (wie Deutsche Sammlung von Mikroorganismen und Zellkulturen GmbH (DSMZ), American Type Culture Collection (ATCC), usw.) in sehr großer Anzahl für das Verfahren benötigte Gene, Vektoren, Organismen, usw. vorhanden. Die für das Verfahren sonst noch benötigten Gene, Vektoren, Enzyme, Proteine, Antibiotika, usw. können kommerziell erworben oder leicht ohne erfinderisches Zutun aus dem Stand der Technik hergestellt werden.

Eine allgemeine Ausführungsform des erfindungsgemäßen Verfahrens sei anhand der Synchronisation einer Kultur eines Mikroorganismus erläutert.

Eine asynchron wachsende Vorkultur des molekularbiologisch veränderten Mikroorganismus wird als Inoculum in einem Kulturgefäß mit einem größeren Volumen eines Mediums gegeben, das zur Substanz-Pulserzeugung entsprechend angepaßt wurde.

Bei der Durchführung des erfindungsgemäßen Verfahrens werden bis auf die Anpassung zur Pulserzeugung die üblichen Kulturbedingungen für den Mikroorganismus eingehalten.

Nach Inoculumzugabe wird der erste Substanz-Puls im Medium erzeugt. Alle Mitglieder der Population, die während der Pulsdauer die Vorstufe der mindestens wachstumsfördende Substanz in die mindestens wachstumsfördende Substanz überführen oder die wachstumshemmende Substanz inaktivieren, werden im Gegensatz zu den anderen Mitgliedern der Population zum weiteren Wachstum gefördert bzw. nicht am weiterem Wachstum behindert und können weiter wachsen. Die anderen Mitglieder werden am weiteren Wachstum behindert oder sterben sogar ab. Im Zeitabstand mindestens eines kompletten Zellzyklus der Mitglieder der Population wird der zweite Substanz-Puls verabreicht. Diejenigen Mitglieder der Population, die während der Pulsdauer und in den Zeitabständen zwischen beiden Substanz-Pulsen die Vorstufe der mindestens wachstumsfördenden Substanz in die mindestens wachstumsfördende Substanz überführen oder die wachstumshemmende Substanz inaktivieren, werden im Gegensatz zu den anderen Mitgliedern der Population zum weiteren Wachstum gefördert bzw. nicht am weiterem Wachstum behindert und können weiter wachsen. Die anderen Mitglieder, die nicht genau synchron zu dem Zeitabstand zwischen den beiden Substanz-Pulsen oder die Substanz nur in anderen Zeitabständen umsetzen können, werden am weiteren Wachstum behindert oder sterben sogar ab. Erst nach dem zweiten Substanz-Puls kann deshalb eine synchronisierte Kultur vorliegen, wenn die anderen Mitglieder abgetötet werden. Werden die anderen Mitglieder der Kultur hingegen nur am Wachstum behindert, findet eine nachweisbare Synchronisation aufgrund der bekannten Populationswachstumsgesetzen erst nach einer gewissen Anzahl von weiteren Substanz-Pulsen statt. In weiteren Zeitabständen mindestens eines kompletten Zellzyklus der Mitglieder der Population werden weitere Substanz-Pulse verabreicht, um die Kultur synchron zu halten. Wird statt der asynchron wachsenden Vorkultur des molekularbiologisch veränderten Mikroorganismus eine Kultur mit dem Transformanten/Transfektantengemisch des Transformations/Transfektionsverfahrens eingesetzt, kann das Verfahren zur Anreicherung und Selektion der für das Verfahren benötigten molekularbiologisch veränderten Mitglieder der Population verwendet werden. Man erhält eine Mischung von geeigneten Transfektanten/Transformanten, deren Populationen zwar jede für sich synchron wächst, die sich aber in unterschiedlichen Zellzyklusabschnitten befinden, und durch Separation getrennt werden müssen.

Die mit der Erfindung erziehen Vorteile bestehen insbesondere darin, daß
- eine direkt an den Zellzyklus der Mitglieder der Population orientierte andauernde Synchronisation erreicht wird,
- in die Physiologie der synchronisierten Mitglieder der Population kaum oder gar nicht eingegriffen wird,
- die üblicherweise eingesetzten Kulturbedingungen, Kulturmedien und Kulturgefäße bis auf die Änderungen für die Erzeugung des Pulses beibehaltenen werden,
- sich unterschiedliche Populationsgrößen synchronisieren lassen,
- sich Populationen, deren Mitglieder Zellhaufen bilden, synchronisieren lassen,
- die Synchronisation für viele verschiedene Mikroorganismen, Organismen, Zellinien, usw. einsetzbar ist,
- bei Mischkulturen von mehreren Populationen eine gemeinsame oder unabhängige Synchronisation der Mitglieder der jeweiligen Population durch geeignete Wahl der eingesetzten Substanzen erreicht wird,
- durch geeignete Wahl von mehreren unterschiedlichen eingesetzten Substanzen eine Synchronisation zu unterschiedlichen Zeitabschnitten und/oder in verschiedenen Seitenzweigen des Zellzyklus derselben Mitglieder der Population erreicht wird,
- das Synchronisationsverfahren durch die Erzeugung einer zellzyklusabhängigen Lumineszenz oder meßbaren Substanz der Mitglieder der Population genau gesteuert werden kann,
- eine gezielte zellzyklusabhängige Umsetzung/Herstellung von Substanzen sich durch zusätzlich übertragene Enzym/Proteingenen bei der Herstellung der für das Verfahren benötigten molekularbiologisch Mitglieder erreichen läßt.

Das erfindungsgemäße Verfahren zur Synchronisation von Zellteilungen einer Kultur oder Mischkultur mit jeweils mindestens zwei Mitgliedern mindestens einer Population ist, neben der Gewinnung von zellzyklussynchronisierten Zellen, für eine zellzyklusabhängig oder zellzyklusabhängig gemachte Herstellung von Substanzen, wie Proteinen, Enzymen, niedermolekularen Verbindungen usw., und/oder für eine zellzyklusabhängige Umsetzung von Substanzen von großem wirtschaftlichem Interesse.

Das erfindungsgemäße Verfahren sei nun an Hand von einigen Beispielen näher erläutert.

### Beispiel 1

Mit diesem Beispiel soll der Einsatz des erfindungsgemäßen Verfahrens zur Selektion und Anreicherung der für das erfindungsgemäße Verfahren benötigten molekularbiologisch modifizierten Mitglieder der Population eines Bakterienstamms gezeigt werden.

Bei der Gewinnung von Proteinasen als Waschmittelzusatz wird gewöhnlich als Proteinasequelle ein *Bacillus*-Stamm und zur Anzucht dieses Mikroorganismus eine asynchron wachsende Kultur in einem bestimmten Medium und in einer Apparatur von unterschiedlichster Größe verwendet.

Der *Bacillus*-Stamm, dessen Kultur synchronisiert werden soll, muß sensitiv gegen eine mindestens wachstumshemmende Substanz sein oder mit den bekannten üblichen Mutationsverfahren sensitiv gegen eine mindestens wachstumshemmende Substanz gemacht worden sein, damit die für das Verfahren benötigten molekularbiologisch modifizierten Mitglieder hergestellt werden können. Diese mindestens wachstumshemmende Substanz soll in diesem Beispiel das kommerziell erhältliche Penicillin G sein. Ebenfalls muß ein Gen für ein Enzym in geeigneter Form für die Transformation oder Transfektion dieses *Bacillus*-Stammes zur Verfügung stehen, dessen exprimiertes Genprodukt diese mindestens wachstumshemmende Substanz inaktiviert. Bei Penicillin G kann zwischen den Enzymen Penicillinase, Penicillin acyl esterase oder Penicillinamidase ausgewählt werden. Eine geeignete Form ist ein Genkonstrukt des gewählten Gens aus einem anderen kommerziell erhältlichen Bakterium oder Vektor flankiert von der oben angegebenen Region eines der oben genannten für den Mikroorganismus passenden G1-Cycline, die auf die PEST-Region folgt, und das ein durchgängiges Leseraster besitzt. Ebenso muß ein Enzym zur Verfügung stehen, das diese mindestens wachstumshemmende Substanz im verwendeten Medium inaktivieren kann. Man kann das gleiche Enzym einsetzen, wie für die Transformation oder Transfektion, oder ein anderes von den oben aufgeführten Enzymen oder ein Gemisch der oben aufgeführten Enzyme. Das Enzym oder die Enzyme können entweder kommerziell erworben sein oder aus anderen Mikroorganismen, die diese Enzyme exprimieren, mit den bekannten Isolationsverfahren gewonnen werden. Das Enzym oder die Enzyme können entweder frei dem üblicherweise verwendeten Medium für diesen *Bacillus*-Stamm zugegeben werden oder aus Stabilitätsgründen usw. an einem Träger gebunden sein.

Zuerst wird das Gen mit einem Transformations/Transfektionsverfahren, wie zum Beispiel mit der Elektroporation oder mit den in US Pat. Nr. 5716807 aufgeführten Verfahren, in den *Bacillus*-Stamm überführt. Unter den Transformanten/Transfektanten werden auch solche sein, die das Gen durch homologe Rekombination an Positionen in ihre genomische DNA integriert haben, an denen das jeweilige G1-Cyclingen des Zellzyklus liegt. Einige dieser Transformanten/Transfektanten werden zum Beispiel ein funktionelles Fusionsprodukt aus dem Zellzyklusgen und dem transformierten Gen exprimieren. Da beim erfindungsgemäßen Verfahren Selektionsbedingungen vorliegen, die Transformanten/Transfektanten mit einer zellzyklusabhängigen Expression des Penicillin G-inaktivierenden Enzyms begünstigen, wird nach der Transformation/Transfektion das erfindungsgemäße Verfahren zur Selektion und Anreicherung der für das erfindungsgemäße Verfahren benötigten molekularbiologisch modifizierten Mitglieder der Population eingesetzt. Mit den üblichen Verfahren zur Selektion und Anreicherung der gewünschten Mitglieder der Population werden zwar die nicht transformierten/transfektierten Bakterienzellen abgetötet oder am Wachstum gehemmt, da aber Selektionsbedingungen bei diesen Verfahren vorliegen, die Transformanten/Transfektanten mit einer nicht zellzyklusabhängigen Expression des Penicillin G-inaktivierenden Enzyms begünstigen, können diese Verfahren nur bedingt oder gar nicht eingesetzt werden. Die Generationszeit des ursprünglich verwendeten *Bacillus*-Stamms im jeweils üblicherweise eingesetzten Medium, den Kulturbedingungen und den verwendeten Kulturgefäßen muß als Richtwert für die weitere Vorgehensweise bekannt sein oder ermittelt werden. Besonders geeignet bei der Selektion und Anreicherung der für das erfindungsgemäße Verfahren benötigten molekularbiologisch modifizierten Mitglieder der Population sind als Kulturgefäße Mikrotiterplatten mit großen Wells oder Multischalen mit kleinen Schalen. Des weiteren werden die minimalen Pulsdauern der Penicillin G-Zugabe mit verschiedenen Penicillin G-Konzentrationen bei verschiedenen dem Medium zusetzten Mengen an Penicillin G-inaktivierenden Enzym oder Enzymen ermittelt, die den ursprünglich verwendeten *Bacillus*-Stamm am Wachstum hindern oder sogar abtöten. Die Pulsdauer bei verschiedenen zugesetzten Penicillin G-Konzentrationen hängt von der dem Medium zugesetzten Menge an Penicillin G-inaktivierenden Enzym oder Enzymen ab, dem üblicherweise verwendeten Medium, den verwendeten Kulturgefäßen und den Kulturbedingungen, und wird deshalb empirisch eingestellt.

Zur Variation der Pulsdauer kann man ferner erstens unterschiedliche Penicillin G-Konzentrationen einsetzen und die Menge des dem Medium zugesetztem Enzyms oder der Enzyme konstant halten oder zweitens eine bestimmte Penicillin G-Konzentration verwenden und unterschiedliche Mengen des dem Medium zugesetztem Enzyms oder der Enzyme einsetzen. Nur der zweite Fall wird in diesem Beispiel durchgeführt. Es wird eine Reihe von Medien mit dem zugesetztem Enzym oder den Enzymen mit verschiedenen Pulsdauern bei der Zugabe einer bestimmten Penicillin G-Konzentration angesetzt, deren Bereich über der minimalen Pulsdauer und unterhalb der ermittelten Generationszeit liegt. Jedes der angesetzten Medien wird in die Wells der Mikrotiterplatten oder die Schalen der Multiplatten in ausreichender Menge und mit ausreichendem Volumen pipettiert. Um Verwechslungen zu vermeiden, empfiehlt es sich, alle Schalen einer Multiplatte oder die Wells einer Mikrotiterplatte nur mit einem Medium zu füllen. Da bei allen Medien der Reihe die Vorgehensweise identisch ist, wird an einem Beispiel die weitere Durchführung besprochen, die sich auch automatisiert läßt. In die erste Schale einer Multischale mit 48 Schalen mit dem Medium, bei dem die Penicillin G-Pulsdauer das Doppelte der minimalen Pulsdauer beträgt, wird ein Teil des erzeugten Transformanten/Transfektantengemisches überführt und anschließend der erste Penicillin G-Puls verabreicht. Nach der Dauer einer Generationszeit wird der zweite Penicillin G-Puls und periodisch in Zeitabständen der Dauer einer Generationszeit weitere Penicillin G-Pulse verabreicht. Sobald eine gewisse Zelldichte erreicht ist, das Medium verbraucht ist oder sich unerwünschte Substanzen im Medium zu stark angehäuft haben, wird ein Aliquot der Kultur z.B. 1/10 bis 1/20 des Kulturvolumens) der ersten Schale entnommen und in die zweite Schale überführt. In der zweiten Schale wird dann die periodische Verabreichung der Penicillin G-Pulse fortgesetzt, bis wiederum eine gewisse Zelldichte erreicht ist, das Medium verbraucht ist, das zugesetzte Enzym in seiner Aktivität sich vermindert oder unerwünschte Substanzen sich im Medium zu stark angehäuft haben. In diesem Fall wird ein Aliquot in die dritte Schale überführt.

Die periodischen Verabreichung der Penicillin G-Pulse und das Überführen jeweils eines Aliquots der Kultur in frisches Medium aus den oben genannten Gründen, wird solange fortgesetzt, bis sich entweder bei dieser Kultur oder bei mindestens einer der Kulturen mit einer anderen Pulsdauer eine synchronisierte Population von Zellen nachweisen läßt. Falls bei keiner Kultur eine Synchronisation nachweisbar ist, weil zum Beispiel ein Gemisch von synchronisierten Transformanten/Transfektanten mit unterschiedlichen Stadien des Zellzyklus vorliegt, führt man das erfindungsgemäße Verfahren solange fort bis eine genügende Generationsanzahl von der Kultur durchlaufen wurde. Die minimalste erforderliche Generationsanzahl kann aus der geschätzten Wahrscheinlichkeit für eine erfolgreiche Transformation/Transfektion, der Anfangskonzentration an Zellen nach Transformation/Transfektion im Medium, einer angenommenen Endkonzentration an geeigneten Transformanten/Transfektanten und der üblichen Generationszeit abgeschätzt werden. Es versteht sich, daß bei der Ausführung des erfindungsgemäßen Verfahrens die optimalen Kulturbedingungen eingehalten werden oder nur eine sehr kurze Abweichung von den geeigneten optimalen Kulturbedingungen verursacht wird. Gegebenenfalls kann man auch periodische Verabreichungen der Penicillin G-Pulse in Vielfachen oder Kombinationen von Vielfachen der bestimmten Generationszeit einsetzen und/oder die Abstände der Penicillin G-Pulse zueinander etwas größer als die bestimmte Generationszeit machen, um besser geeignete Transformanten/Transfektanten zu erlangen. Da bei einer Kultur mit einer sehr kurzen Penicillin G-Pulsdauer nahe der minimalen Penicillin G-Pulsdauer besonders günstige Selektionsbedingungen vorliegen für die Selektion und Anreicherung von Transformanten/Transfektanten mit einer zellzyklusabhängigen Expression des Penicillin G-inaktivierenden Enzyms und auch Transformanten/Transfektanten mit besseren Synchronisationsgraden als bei Kulturen mit längerandauernden Penicillin G-Pulsen zu erwarten sind, werden zuerst die Kulturen mit den kürzesten Penicillin G-Pulsdauern nach geeigneten einzelnen Transformanten/Transfektanten gescreent. Die Transformanten/Transfektanten werden dazu auf Petrischalen mit den üblicherweise verwendeten festen Nährböden ohne Zusatz von Penicillin G ausgespatelt und wachsen gelassen. Nach Vermehrung einzelner Zellen zu Kolonien können diese separiert werden und vermehrt werden. Zum Screening nach denjenigen Transformanten/Transfektanten, die zum Beispiel ein Fusionsprodukt des übertragenen Gens mit einem zellzyklusabhängigen Gen besitzen, kann die Polymerasekettenreaktion oder die Hybridisierung mit spezifischen Sonden eingesetzt werden. Alternativ kann mit Durchflußzytometrie-Verfahren nach Transformanten/Transfektanten gesucht werden, die eine zellzyklusabhängige Expression des übertragenen Gens zeigen (z.B.: Eine gewisse Zellgröße und Expressionsstärke des Gens zeigen eine Korrelation zueinander).

### Beispiel 2

Bei diesem Beispiel soll der Einsatz des erfindungsgemäßen Verfahrens zur Selektion und Anreicherung der für das erfindungsgemäße Verfahren benötigten molekularbiologisch modifizierten Mitglieder der Population von adhärenten humanen Zellinien, die keine Feederzellen benötigen, und bei der anschließenden Synchronisation im Labor- und Technikmaßstab gezeigt werden.

Humane Zellinien werden in unterschiedlichsten Bereichen, wie zum Beispiel in der Produktion von humanen Proteinen (z.B.: Albumin, Caeruloplasmin), in der Herstellung von Diagnostika (z.B.: Zellen zum Nachweis von Antiköper gegen Kernbestandteile, Chromosomengewinnung, usw.), Kultivierung von Viren, Herstellung von Vaccinen usw. eingesetzt. Humane Zellinien benötigen zum Wachstum unterschiedlichste Substanzen, wie Isoleucin, Leucin und Valin, um nur einige zu nennen. Als Ausführungsart des erfindungsgemäßen Verfahrens bietet sich daher an, eine pulsartige Zugabe der Vorstufe von Isoleucin, Leucin, Valin oder einer anderen essentiellen Aminosäure einzusetzen, die erst durch ein geeignetes eingebrachtes zellzyklusabhängiges Enzym in die entsprechende essentielle Aminosäure umgesetzt wird.

Homocystein, die Vorstufe von Methionin, kann allerdings nur bei humanen Zellinien eingesetzt werden, die keine funktionelle 5-Methyltetrahydrofolat:Homocysteinmethyltransferase besitzen.

Im weiteren soll am Beispiel des Einsatzes von α-Keto-β-methylvalerat, der Vorstufe bei der Biosynthese von Isoleucin in Bakterien, und der adhärent wachsenden humanen Zellinie Hep G2 (siehe US Pat Nr. 4393133 für Kulturbedingungen usw.) die weitere Durchführung besprochen werden. Um die Vorstufe wieder aus dem Medium entfernen zu können, kann das Enzym α-Ketoisovalerat-Dehydrogenase, das auch α-Ketoisovalerat (Biosynthesevorstufe für Valin in Bakterien) und α-Ketoisocaproat (Biosynthesevorstufe für Leucin in Bakterien) umsetzt, als Zusatz des Mediums in freier oder gebundener Form an einem Träger zur Verwendung kommen. Die jeweils beste Darreichungsform des Enzyms muß durch Vorversuche ermittelt werden. Dieses Enzym benötigt außerdem als weitere Substrate NAD⁺ und Coenzym A im Medium. Da die üblicherweise eingesetzten Medien Isoleucin enthalten, muß das Medium zunächst von Isoleucin befreit werden mit einem der bekannten Verfahren (z.B.: T. Lindl und J. Bauer. Zell- und Gewebekultur. Gustav Fischer Verlag, 2. Auflage, Stuttgart, 1989: 188; P. Fantes und R. Brooks. The cell cycle: a practical approach. IRL press at Oxford university press, Oxford, 1993: 4-5). Das bakterielle Gen des Enzyms Valin-Aminotransferase kommt als Gen in Betracht, um in geeigneter Form in die Zellinie transformiert zu werden (zum Beispiel mittels direkter Transformation: Eine geeignete Form ist ein Restriktionsfragment des kompletten Gens), da dieses Enzym α-Keto-β-methylvalerat in Isoleucin und α-Ketoisovalerat in Valin umsetzt. Dieses Enzym benötigt als Substrate zusätzlich noch Glutamat und Pyridoxal-5-phosphat, die humane Zellinien in den meisten Fällen schon intrazellulär in ausreichenden Mengen besitzen.

### (1. Selektion und Anreicherung der benötigten molekularbiologisch modifizierten Mitglieder der Population durch das erfindungsgemäße Verfahren)

Da beim erfindungsgemäßen Verfahren, wie im Beispiel 1 erwähnt, Selektionsbedingungen vorliegen, die Transformanten mit einer zellzyklusabhängigen Expression des übertragenen Enzyms begünstigen, wird wie im Beispiel 1 nach der direkten Transformation das erfindungsgemäße Verfahren zur Selektion und Anreicherung der für das erfindungsgemäße Verfahren benötigten molekularbiologisch modifizierten Mitglieder der Population eingesetzt.

Aus den in Beispiel 1 genannten Gründen können die üblicherweise bekannten Verfahren zur Selektion und Anreicherung der für das erfindungsgemäße Verfahren benötigten molekularbiologisch modifizierten Mitglieder der Population nur bedingt oder gar nicht eingesetzt werden. Die Generationszeit der ursprünglich verwendeten Hep G2-Zellinie im jeweils üblicherweise eingesetzten Medium, den Kulturbedingungen und den verwendeten Kulturgefäßen muß als Richtwert für die weitere Vorgehensweise bekannt sein oder ermittelt werden. Besonders geeignet bei der Selektion und Anreicherung der für das erfindungsgemäße Verfahren benötigten molekularbiologisch modifizierten Mitglieder der Population sind als Kulturgefäße Mikroschalen. Die Pulsdauer bei verschiedenen zugesetzten α-Keto-β-methylvalerat-Konzentrationen hängt von der dem Medium zugesetzten Menge an α-Ketoisovalerat-Dehydrogenase, dem verwendeten Medium mit den beigefügten Substanzen (NAD⁺, Coenzym A, evtl. Glutamat, evtl. Pyridoxal-5-phosphat), den verwendeten Kulturgefäßen und den Kulturbedingungen ab, und wird deshalb experimentell eingestellt. Zur Variation der Pulsdauer kann man wahlweise erstens unterschiedliche α-Keto-β-methylvalerat-Konzentrationen einsetzen und die Menge des dem Medium zugesetztem Enzyms konstant halten oder zweitens eine bestimmte α-Keto-β-methylvalerat-Konzentration verwenden und unterschiedliche Mengen des dem Medium zugesetztem Enzyms einsetzen. Eine Reihe von Medien mit dem zugesetztem Enzym mit verschiedenen Pulsdauern bei Zugabe einer bestimmten α-Keto-β-methylvalerat -Konzentration wird angesetzt, deren Bereich über beispielsweise 1/20 der ermittelten Generationszeit und unterhalb der ermittelten Generationszeit liegt. Da der Bedarf der unterschiedlichen Transformanten im Gemisch an der essentiellen Aminosäure differiert, kann die jeweilige minimale Pulsdauer nicht bestimmt werden. Die 1/20 der ermittelten Generationszeit als kleinste Pulsdauer stellen deshalb nur ein Richtwert dar. Kürze Pulsdauern könnten selbstverständlich auch durchgeführt werden. Jedes der angesetzten Medien wird nun in die Schalen der Multischalen in ausreichender Menge und mit ausreichendem Volumen pipettiert. Um Verwechslungen zu vermeiden, empfiehlt es sich, alle Schalen einer Multischale nur mit einem Medium zu füllen. Da bei allen Medien der Reihe die Vorgehensweise identisch ist, wird an einem Beispiel die weitere Durchführung besprochen. In die erste Schale einer Multischale mit 48 Schalen mit dem Medium, bei dem die Pulsdauer 1/10 der Generationszeit beträgt, wird ein Teil der erzeugten Transformanten pipettiert und anschließend der erste α-Keto-β-methylvalerat-Puls verabreicht. Nach der Dauer einer Generationszeit wird der zweite α-Keto-β-methylvalerat-Puls und periodisch in Zeitabständen der Dauer einer Generationszeit werden weitere α-Keto-β-methylvalerat-Pulse verabreicht. Sobald eine gewisse Zelldichte von adhärenten Zellen auf dem Schalenboden erreicht ist, das Medium verbraucht ist, das zugesetzte Enzym in seiner Aktivität sich vermindert oder sich unerwünschte Substanzen im Medium zu stark angehäuft haben, wird ein abtrypsiniertes Aliquot der Zellen (z.B.: 10% bis 30%) der ersten Schale entnommen. Die Zellen werden kurz abzentrifugiert und der Überstand mit dem Trypsin und anderen Substanzen wird verworfen. Die Zellen werden in frisches Medium aufgenommen und in die zweite Schale überführt. In der zweiten Schale wird dann die periodische Verabreichung der α-Keto-β-methylvalerat-Pulse fortgesetzt, bis wiederum eine gewisse Zelldichte von adhärenten Zellen auf dem Schalenboden erreicht ist, das Medium verbraucht ist oder unerwünschte Substanzen sich im Medium zu stark angehäuft haben. In diesem Fall wird ein abtrypsiniertes Aliquot der Zellen der zweiten Schale entnommen und zentrifugiert. Der Überstand wird verworfen, die Zellen in frisches Medium aufgenommen und in die dritte Schale überführt. Die periodischen Verabreichung der α-Keto-β-methylvalerat-Pulse und das Überführen jeweils eines Aliquots von Zellen in frisches Medium aus den oben genannten Gründen wird solange fortgesetzt, bis sich entweder bei dieser Kultur oder bei mindestens einer der Kulturen mit einer anderen α-Keto-β-methylvalerat-Pulsdauer eine synchronisierte Population von Zellen nachweisen läßt. Falls aus den in Beispiel 1 genannten Gründen bei keiner Kultur eine Synchronisation nachweisbar ist, führt man das erfindungsgemäße Verfahren solange fort bis eine genügende Generationsanzahl (siehe Beispiel 1) von der Kultur durchlaufen wurde. Es versteht sich, daß bei der Ausführung des erfindungsgemäßen Verfahrens die optimalen Kulturbedingungen eingehalten werden oder nur eine sehr kurze Abweichung von den optimalen Kulturbedingungen verursacht wird.

Gegebenenfalls kann man auch periodische Verabreichungen der α-Keto-β-methylvalerat-Pulse in Vielfachen oder Kombinationen von Vielfachen der bestimmten Generationszeit einsetzen und/oder die Abstände der α-Keto-β-methylvalerat-Pulse zueinander etwas größer als die bestimmte Generationszeit machen, um besser geeignete Transformanten zu erlangen. Da die Hep G2-Zellinie eine adhärent wachsende Zellinie ist, kann die obige Durchführung des erfindungsgemäßen Verfahrens nach Prüfung auf Eignung vereinfacht werden. Man setzt mehrere Medien an, erstens isoleucinfreies Medium und zweitens isoleucinfreie Medien mit verschiedenen α-Keto-β-methylvalerat-Konzentrationen. Ein Abbau des α-Keto-β-methylvalerat im Medium ist bei der Vereinfachung nicht mehr erforderlich. Der α-Keto-β-methylvalerat-Puls kann durch Austausch von Medium ohne α-Keto-β-methylvalerat und eines der Medien mit α-Keto-β-methylvalerat erzeugt werden, wobei verschiedene Pulsdauer und verschiedene Konzentrationen kombiniert werden können. Da bei allen Kombinationen die Vorgehensweise identisch ist, wird an einem Beispiel die weitere Durchführung besprochen. In die erste Schale der Mikroschale wird das Medium ohne Isoleucin in ausreichender Menge und ein Teil des erzeugten Transformantengemisches pipettiert. Nachdem die Zellen sich am Schalenboden festgesetzt haben, wird dieses Medium sofort ohne die Zellen abzulösen vorsichtig entfernt und durch eines der Medien mit einer bestimmten α-Keto-β-methylvalerat-Konzentration ersetzt. Nach einer Zeitdauer von beispielsweise 1/10 der Generationszeit wird das zweite Medium wieder durch das erste Medium ersetzt, wodurch ein α-Keto-β-methylvalerat-Puls erzeugt wurde. Die benutzten Medien sollten nach Entfernung von Zellen, die sich trotz vorsichtiger Durchführung abgelöst haben, soweit es geht bevorzugt wiedereingesetzt werden, da bekannt ist, daß humane Zellinien selbst zum Beispiel Wachstumsstoffe produzieren, die sie ins Medium abgeben. Nach der Dauer einer Generationszeit wird der zweite α-Keto-β-methylvalerat-Puls wiederum durch einfachen Austausch der beiden Medien erreicht. Bis auf diese Änderung der Durchführung ist das Verfahren wie oben weiter auszuführen. Die Kulturen mit den kürzesten α-Keto-β-methylvalerat-Pulsdauern und den geringsten α-Keto-β-methylvalerat-Konzentrationen werden aufgrund der in Beispiel 1 genannten Gründe zuerst nach geeigneten einzelnen Transformanten gescreent. Die Transformanten der einzelnen Kulturen werden mit den bekannten Verfahren separiert und vermehrt. Zum Screening nach denjenigen Transformanten, die zum Beispiel ein Fusionsprodukt des übertragenen Gens mit einem zellzyklusabhängigen Gen besitzen, kann die Polymerasekettenreaktion oder die Hybridisierung mit spezifischen Sonden eingesetzt werden.

Alternativ kann mit Durchflußzytometrie-Verfahren nach Transformanten gesucht werden, die eine zellzyklusabhängige Expression des übertragenen Gens zeigen.

### (2. Anpassung des erfindungsgemäßen Verfahren auf Labor- und Technikmaßstab)

Um das erfindungsgemäße Verfahren in den beiden oben beschriebenen Durchführungsformen bei der Hep G2-Zellinie auch im Labor- und Technikmaßstab durchzuführen zu können, muß die pulsartige Verabreichung der Vorstufe im eingesetzten Medium auf die größeren Kulturgefäße und Kulturvolumina angepaßt werden, da es durch die veränderte Geometrie sein kann, daß die bisherige Pulsdauer nicht mehr dazu ausreicht, daß die Mitglieder der synchronisierten Population in ausreichender Menge Isoleucin für sich bilden können. Nur die Anpassung der einfachen Durchführungsform wird weiter beschrieben. Durch die vorher durchgeführte Selektion, Anreicherung und der anschließenden Isolation von geeigneten molekularbiologisch modifizierten Mitgliedern der Population ist mindestens die Pulsdauer, die α-Keto-β-methylvalerat-Konzentration, die Abstände zwischen den α-Keto-β-methylvalerat-Pulsen für ein Kulturvolumen bekannt, welche als Richtwerte für das weitere Vorgehen dienen. Das Doppelte dieses Volumens an isoleucinfreies Medium wird in einer Zellkulturflasche vorgelegt und die Zellen der aufgrund des Screeningverfahrens als für geeignet befundenen Transformante zugegeben. Nachdem die Zellen sich auf dem Boden festgesetzt haben, tauscht man das Medium gegen das isoleucinfreies Medium mit α-Keto-β-methylvalerat aus. Nach der bekannten Zeitdauer wird dieses Medium wieder durch das isoleucinfreie Medium ersetzt, wodurch ein α-Keto-β-methylvalerat-Puls erzeugt wird. Nach dem bekannten Zeitabstand zwischen den α-Keto-β-methylvalerat-Pulsen wird der zweite α-Keto-β-methylvalerat-Puls wiederum durch einfachen Austausch der beiden Medien und periodisch in den bekannten Zeitabständen werden weitere α-Keto-β-methylvalerat-Pulse verabreicht. Sobald eine gewisse Zelldichte von adhärenten Zellen auf dem Boden erreicht ist, das Medium verbraucht ist oder sich unerwünschte Substanzen im Medium zu stark angehäuft haben, wird ein abtrypsiniertes Aliquot der Zellen (z.B.: 10% bis 30%) der Zellkulturflasche entnommen. Die Zellen werden kurz abzentrifugiert und der Überstand mit demTrypsin und anderen Substanzen wird verworfen. Die Zellen werden in frisches Medium aufgenommen und in eine andere Zellkulturflasche überführt, wobei die Übertragung zwischen zwei α-Keto-β-methylvalerat-Pulsen erfolgen sollte. Wenn feststeht, daß die Zellen sich bei diesem größeren Kulturvolumen wie gewohnt teilen, kann das Kulturvolumen um eine weitere Stufe (zum Beispiel auf das Doppelte) vergrößert werden. Falls die Zellen sich nicht wie gewohnt teilen, reicht eventuell die bisherige Zeitdauer der verabreichten α-Keto-β-methylvalerat-Pulsen nicht mehr aus, damit die Zellen der synchronisierten Population in ausreichender Menge Isoleucin für sich bilden können. In diesem Fall wird die Pulsdauer, die α-Keto-β-methylvalerat-Konzentration und der Zeitabstand zwischen den α-Keto-β-methylvalerat-Pulsen soweit vorsichtig verändert, bis die Zellen wie gewohnt wachsen. Wenn das nächst größere Kulturvolumen nicht mehr geeignet ist für die verwendete Zellkulturflasche, wird die Synchronisation in der nächst größeren Zellkulturflasche weiter durchgeführt. Wenn feststeht, daß die Zellen sich in dieser größeren Zellkulturflasche wie gewohnt teilen, kann auch das Kulturvolumen vergrößert werden. Ansonsten ist wie oben beschrieben zu verfahren. Die obige Vorgehensweise zur Anpassung ist solange fortzusetzen, bis die synchronisierte Kultur ins gewünschten Kulturgefäß und ins gewünschte Kulturvolumen überführt worden ist. Bei großen Kulturvolumen muß man gegebenenfalls die Art der Erzeugung des α-Keto-β-methylvalerat-Pulses auf eine andere Art der Erzeugung umstellen, wenn die bisherige Erzeugung ab einem gewissen Kulturvolumen Probleme bereitet.

### Beispiel 3

Bei diesem Beispiel soll der Einsatz des erfindungsgemäßen Verfahrens bei der Selektion, der Anreicherung und beim Screening des für das erfindungsgemäße Verfahren benötigten molekularbiologisch modifizierten Bakteriums *Gluconobacter oxydans* ssp. *suboxydans* und die anschließende Anpassung des erfindungsgemäßen Verfahrens mit Hilfe der ebenfalls übertragenen Firefly Luciferase auf Labor- und Technikmaßstab gezeigt werden.

Für die Umsetzung von D-Sorbit zu L-Sorbose, einer Vorstufe für die chemische Synthese von L-Ascorbinsäure, und für andere Oxidationsreaktionen wird industriell eine Subspecies von *Gluconobacter oxydans* ssp. *suboxydans* (z.B.: US Pat. Nr. 4246345 mit Angabe der Kulturbedingungen) und zur Anzucht dieses Mikroorganismus eine asynchron wachsende Kultur in einem bestimmten Medium und in einer Apparatur von unterschiedlichster Größe verwendet.

Ein Kanamycin A-sensitiver bzw. Kanamycin A-senitiv gemachter *Gluconobacter oxydans* ssp. *suboxydans* soll in diesem Beispiel der Mikroorganismus sein, der für das erfindungsgemäße Verfahren molekularbiologisch modifiziert wird. Die mindestens wachstumshemmende Substanz ist in diesem Fall Kanamycin A. Eine geeignete Form für die Transformation/Transfektion ist ein Genkonstrukt aus dem Gen für die Neomycinphosphotransferase, die Kanamycin A, Neomycin und Streptomycin inaktiviert, und dem Gen für die Firefly Luciferase, wobei die beiden Gene flankiert sind von der oben angegebenen der PEST-Region folgenden Region eines der oben genannten für den Mikroorganismus passenden G1-Cycline, und das ein durchgängiges Leseraster besitzt. Dieses Genkonstrukt kann mit den bekannten molekularbiologischen Verfahren hergestellt werden. Um Kanamycin A im Medium inaktivieren zu können, wird das Enzym Neomycinphosphotransferase eingesetzt, das noch Acetyl-CoA als Substrat benötigt.

Das Enzym kann entweder kommerziell erworben sein oder aus anderen Mikroorganismen, die dieses Enzym exprimieren, mit den bekannten Isolationsverfahren gewonnen werden. Das Enzym kann entweder frei dem üblicherweise verwendeten Medium für *Gluconobacter oxydans* ssp. *suboxydans* zugesetzt oder aus Stabilitätsgründen usw. an einem Träger gebunden sein.

### (1. Selektion und Anreicherung der benötigten molekularbiologisch modifizierten Mitglieder der Population durch das erfindungsgemäße Verfahren)

Die Durchführung des erfindungsgemäßen Verfahrens zur Selektion und Anreicherung des für das erfindungsgemäße Verfahren benötigten molekularbiologisch modifizierten *Gluconobacter oxydans* ssp. *suboxydans* erfolgt auf analoge Weise wie beim Beispiel 1. Zuerst wird das hergestellte Fusionsprodukt der Gene mit einem Transfektionsverfahren, wie zum Beispiel mit der Elektroporation, überführt. Unter den Transfektanten werden auch solche sein, die das Fusionsprodukt durch homologe Rekombination an Positionen in ihre genomische DNA integriert haben, an den das jeweilige G1-Cyclingen liegt. Einige dieser Transfektanten werden zum Beispiel ein funktionelles Genprodukt aus dem Zellzyklusgen, des Neomycinphosphotransferasegens und des Gens für die Firefly Luciferase exprimieren. Die Generationszeit vom ursprünglich verwendeten *Gluconobacter oxydans* ssp. *suboxydans* im jeweils üblicherweise eingesetzten Medium, den Kulturbedingungen und den verwendeten Kulturgefäßen muß als Richtwert für die weitere Vorgehensweise bekannt sein oder ermittelt werden. Des weiteren werden die minimalen Pulsdauern der Kanamycin A-Zugabe mit verschiedenen Kanamycin A-Konzentrationen bei verschiedenen dem Medium zusetzten Mengen an Neomycinphosphotransferase ermittelt, die den ursprünglich verwendeten *Gluconobacter oxydans* ssp. *suboxydans* am Wachstum hindern oder sogar abtöten. Die Pulsdauer bei verschiedenen zugesetzten Kanamycin A-Konzentrationen hängt von der dem Medium zugesetzten Menge an Neomycinphosphotransferase, dem üblicherweise verwendeten Medium mit dem zugesetzten Substrat für die Neomycinphosphotransferase, den verwendeten Kulturgefäßen und den Kulturbedingungen ab, und wird deshalb empirisch eingestellt. Zur Variation der Pulsdauer kann man wahlweise erstens unterschiedliche Kanamycin A-Konzentrationen einsetzen und die Menge des dem Medium zugesetztem Enzyms konstant halten oder zweitens eine bestimmte Kanamycin A-Konzentration verwenden und unterschiedliche Mengen des dem Medium zugesetztem Enzyms einsetzen. Es wird eine Reihe von Medien mit dem zugesetztem Enzym Neomycinphosphotransferase mit verschiedenen Pulsdauern bei der Zugabe einer bestimmten Kanamycin A-Konzentration angesetzt, deren Bereich über der minimalen Pulsdauer und unterhalb der ermittelten Generationszeit liegt. Jedes der angesetzten Medien wird in die Schalen von Multiplatten mit 48 Schalen in ausreichender Menge und mit ausreichendem Volumen pipettiert. Um Verwechslungen zu vermeiden, empfiehlt es sich, alle Schalen einer Multiplatte nur mit einem Medium zu füllen. Da bei allen Medien der Reihe die Vorgehensweise identisch ist, wird an einem Beispiel die weitere Durchführung besprochen. In die erste Schale einer Multischale mit dem Medium, bei dem die Kanamycin A-Pulsdauer das Vierfache der minimalen Pulsdauer beträgt, wird ein Teil des erzeugten Transfektantengemisches überführt und anschließend der erste Kanamycin A-Puls verabreicht. Nach der Dauer einer Generationszeit wird der zweite Kanamycin A-Puls und periodisch in Zeitabständen der Dauer einer Generationszeit weitere Kanamycin A-Pulse verabreicht. Sobald eine gewisse Zelldichte erreicht ist, das Medium verbraucht ist oder sich unerwünschte Substanzen im Medium zu stark angehäuft haben, wird ein Aliquot der Kultur (z.B. 1/10 bis 1/20 des Kulturvolumens) der ersten Schale entnommen und in die zweite Schale überführt. In der zweiten Schale wird dann die periodische Verabreichung der Kanamycin A-Pulse fortgesetzt, bis wiederum eine gewisse Zelldichte erreicht ist, das Medium verbraucht ist, das zugesetzte Enzym in seiner Aktivität sich vermindert oder unerwünschte Substanzen sich im Medium zu stark angehäuft haben. In diesem Fall wird ein Aliquot in die dritte Schale überführt.

Die periodischen Verabreichung der Kanamycin A-Pulse und das Überführen jeweils eines Aliquots der Kultur in frisches Medium aus den oben genannten Gründen, wird solange fortgesetzt, bis sich entweder bei dieser Kultur oder bei mindestens einer der Kulturen mit einer anderen Kanamycin A-Pulsdauer eine synchronisierte Population von Zellen nachweisen läßt.

Falls bei keiner Kultur eine Synchronisation nachweisbar ist, weil zum Beispiel ein Gemisch von synchronisierten Transfektanten mit unterschiedlichen Stadien des Zellzyklus vorliegt, führt man das erfindungsgemäße Verfahren solange fort bis eine genügende Generationsanzahl (siehe Beispiel 1) von der Kultur durchlaufen wurde. Es versteht sich, daß bei der Ausführung des erfindungsgemäßen Verfahrens die optimalen Kulturbedingungen eingehalten werden oder nur eine sehr kurze Abweichung von den geeigneten optimalen Kulturbedingungen verursacht wird.

Gegebenenfalls kann man auch periodische Verabreichungen der Kanamycin A-Pulse in Vielfachen oder Kombinationen von Vielfachen der bestimmten Generationszeit einsetzen und/oder die Abstände der Kanamycin A-Pulse zueinander etwas größer als die bestimmte Generationszeit machen, um besser geeignete Transfektanten zu erlangen. Da bei einer Kultur mit einer sehr kurzen Kanamycin A-Pulsdauer nahe der minimalen Kanamycin A-Pulsdauer besonders günstige Selektionsbedingungen vorliegen für die Selektion und Anreicherung von Transfektanten mit einer zellzyklusabhängigen Expression der Neomycinphosphotransferase und auch Transfektanten mit besseren Synchronisationsgraden als bei Kulturen mit längerandauernden Kanamycin A-Pulsen zu erwarten sind, werden zuerst die Kulturen mit den kürzesten Kanamycin A-Pulsdauern nach geeigneten einzelnen Transfektanten weiter selektiert und gescreent.

### (2. Selektion und Screening der benötigten molekularbiologisch modifizierten Mitglieder der Population mit dem erfindungsgemäßen Verfahren)

Zur weiteren Selektion und Screening auf Petrischalen mit festem Medium muß das erfindungsgemäße Verfahren angepaßt werden. Dazu werden Petrischalen mit festem Medien, die unterschiedliche Mengen an Neomycinphosphotransferase enthalten, hergestellt. Die Pulsdauer bei verschiedenen aufgebrachten Kanamycin A-Konzentrationen hängt von der dem festem Medium zugesetzten Menge an Neomycinphosphotransferase und seines Substrates Acetyl-CoA, dem üblicherweise verwendeten festem Medium, den verwendeten Kulturgefäßen und den Kulturbedingungen ab, und wird deshalb experimentell eingestellt. Die minimalen Pulsdauern der Kanamycin A-Zugabe wird mit verschiedenen Kanamycin A-Konzentrationen bei verschiedenen dem festen Medium zugesetzten Mengen an Neomycinphosphotransferase ermittelt, die den ursprünglich verwendeten *Gluconobacter oxydans* ssp. *suboxydans* am Wachstum hindern oder sogar abtöten. Das angereicherte Transfektantengemisch wird zunächst auf Petrischalen mit den üblicherweise verwendeten festen Nährböden gespatelt und wachsen gelassen, bis sich die einzelnen Zellen zu Kolonien entwickelt haben. Durch Volumenmessung der Kolonien und der Zelldichtemessung in einzelnen Kolonien zu unterschiedlichen Zeitpunkten wird als Richtwert die für das erfindungsgemäße Verfahren benötigte Generationszeit von *Gluconobacter oxydans* ssp. *suboxydans* auf dem festen Medium bestimmt, die sich in der Regel von der Generationszeit im flüssigen Medium unterscheidet. Von jeder Petrischale wird eine Replikaplatierung auf eine Petrischale mit dem üblichen festen Nährboden durchgeführt.

Nachdem sich Kolonien bei diesen Petrischalen gebildet haben, wird ein Test der Luciferaseexpression der Zellen der Kolonien auf den Petrischalen nach der Testbeschreibung von F. F. Craig, A. C. Simmonds, D. Watmore, F. McCapra und M. R. H. White (Membrane-permeable luciferin esters for assay of firefly luciferase in live intact cells. Biochem J., 1991, **276**, 637-641) durchgeführt. Transfektanten, die eine funktionelle Luciferase exprimieren, geben sich durch Leuchten der jeweiligen Kolonie zu erkennen und werden gekennzeichnet. Mittels einer besonders empfindlichen CCD-Kamera (z.B.: Wright Instruments, Enfield, Middlesex U.K) werden die nur sehr schwach leuchtenden Kolonien ermittelt und ebenfalls gekennzeichnet. Bei den Petrischalen, bei denen keine Kolonie auch nur ein schwaches Leuchten zeigt, wird die dazugehörige Ausgangsplatte verworfen. Von den übrigen Ausgangsplatten werden entweder mittels Replikaplatierung diesmal die Kolonien auf Petrischalen mit den verschiedenen Neomycinphosphotransferasemengen enthaltenen festem Nährboden übertragen oder, wenn jeweils nur bei wenigen Kolonien eine Lumineszenz nachgewiesen wurde, aus Kostengründen die jeweiligen markierten Kolonien zunächst auf Petrischalen mit dem üblicherweise verwendeten festem Medium einzeln übertragen. Im zweiten Fall werden nach Auswachsen der übertragenen Zellen zu Kolonien mittels Replikaplatierung die Kolonien auf Petrischalen mit festem Nährboden mit den verschiedenen Neomycinphosphotransferasemengen überführt. Da bei allen Petrischalen mit festem Nährboden mit den verschiedenen Neomycinphosphotransferasemengen die Vorgehensweise identisch ist, wird an einem Beispiel die weitere Durchführung besprochen. Wenn die übertragenen Zellen zu gerade sichtbaren Kolonien ausgewachsen sind, wird eine Kanamycin A-Konzentration aufgebracht, die einen Kanamycin A-Puls von 1/10 der ermittelten Generationszeit erzeugt. In Abständen der ermittelten Generationszeit werden weitere Kanamycin A-Pulse verabreicht. Das Wachstum der markierten Kolonien wird verfolgt. Nachdem einige dieser Kolonien deutlich größer geworden sind, wird ein Test der Luciferaseexpression der Zellen der Kolonien auf den Petrischalen wie oben beschrieben durchgeführt, wobei die Bestimmungszeit bei jeder Petrischale über mindestens eine Generationszeit liegen muß. Nur diejenigen markierten Kolonien, die größer geworden sind, werden beim weiteren Screening weiter beobachtet. Von diesen Kolonien leuchten diejenigen Kolonien, die eine zellzyklusabhängige Luciferaseaktivität besitzen, innerhalb der Generationszeit kurzfristig mit einer gewissen Stärke. Da das Aufleuchten sehr schwach sein kann, werden wieder mittels einer besonders empfindlichen CCD-Kamera die nur sehr schwach kurzfristig leuchtenden Kolonien ermittelt. Die ermittelten Kolonien werden von den Ausgangsplatten separiert und weiter vermehrt. Zum weiteren Screening nach denjenigen Transfektanten, die zum Beispiel ein Fusionsprodukt des übertragenen Konstrukts aus zwei Genen mit einem zellzyklusabhängigen Gen besitzen, kann die Polymerasekettenreaktion oder die Hybridisierung mit spezifischen Sonden eingesetzt werden. Alternativ kann mit Durchflußzytometrie-Verfahren nach Transfektanten gesucht werden, die eine zellzyklusabhängige Expression der übertragenen Gene zeigen (z.B.: Eine gewisse Zellgröße und die Firefly Luciferase-Aktivität zeigen eine Korrelation zueinander).

### (3. Anpassung des erfindungsgemäßen Verfahren auf Labor- und Technikmaßstab)

Bei der Anpassung des erfindungsgemäßen Verfahrens auf Labor- und Technikmaßstab wird die periodische Firefly Luciferase-Aktivität zur Ermittlung der jeweils optimalen Pulsdauer und den besten Abständen zwischen den Kanamycin A-Pulsen eingesetzt. Durch die vorher durchgeführte Selektion, Anreicherung, Screening und der anschließenden Isolation von geeigneten molekularbiologisch modifizierten Mitgliedern der Population ist mindestens die Pulsdauer, die Kanamycin A-Konzentration, die Neomycinphosphotransferase-Konzentration im Medium, die Abstände zwischen den Kanamycin A-Pulsen für ein Kulturvolumen bekannt, welche als Richtwerte für das weitere Vorgehen dienen. Das Doppelte dieses Volumens an Medium mit der bekannten Neomycinphosphotransferase-Konzentration wird in einem Kulturgefäß vorgelegt, die Zellen der aufgrund des Screeningverfahrens als für geeignet befundenen Transfektante zugegeben und anschließend der erste Kanamycin A-Puls verabreicht. Nach der bekannten Zeitdauer wird der zweite Kanamycin A-Puls und periodisch in den bekannten Zeitabständen weitere Kanamycin A-Pulse verabreicht. Sobald eine gewisse Zelldichte im Medium erreicht ist, das Medium verbraucht ist oder sich unerwünschte Substanzen im Medium zu stark angehäuft haben, wird ein Aliquot der Zellen (z.B.: 5% bis 10%) dem Kulturgefäß entnommen und in ein anderes Kulturgefäß mit frischem Medium überführt, wobei die Übertragung zwischen zwei Kanamycin A-Pulsen erfolgen sollte. Der Kultur wird zu unterschiedlichen Zeiten Proben entnommen und die Aktivität der Luciferase durch ein Luciferin/Luciferase-Test bestimmt. Die Pulsdauer und die Zeitabstände zwischen den Kanamycin A-Pulsen wird nun solange leicht variiert, bis die bestimmte zeitliche Kurve der periodischen Luciferase-Aktivität möglichst eine maximale zellzahlnormierte Stärke besitzt und einen minimalen Zeitraum einnimmt. Das Kulturvolumen wird dann um eine weitere Stufe (zum Beispiel auf das Doppelte) vergrößert, oder wenn das nächst größere Kulturvolumen nicht mehr geeignet ist für das verwendete Kulturgefäß, wird die Synchronisation in dem nächst größeren Kulturgefäß weiter durchgeführt. Die obige Vorgehensweise zur Anpassung ist solange fortzusetzen, bis die synchronisierte Kultur ins gewünschte Kulturgefäß und ins gewünschte Kulturvolumen überführt worden ist. Die weitere Synchronisation kann dort durch weitere Probennahmen zur Bestimmung der Luciferase-Aktivität laufend überwacht werden, was auch automatisiert werden kann. Bei großen Kulturvolumen muß man gegebenenfalls die Art der Erzeugung des Kanamycin A-Pulses auf eine andere Art der Erzeugung umstellen, wenn die bisherige Erzeugung ab einem gewissen Kulturvolumen Probleme bereitet.

### Beispiel 4

Bei diesem Beispiel soll der Einsatz des erfindungsgemäßen Verfahrens bei der Gewinnung einer zellzyklusabhängige exprimierten Substanz bei einer Sproßhefe im Labor- und Technikmaßstab gezeigt werden.

Physiologische Rassen von *Saccharomyces cerevisiae* werden nicht nur als Brau- und Bäckerhefen eingesetzt, sondern auch bei der Produktion von Invertase und Ethanol. Des weiteren dient *Saccharomyces cerevisiae* als Wirt zur Expression von Fremdgenen für die Produktion von bestimmten Stoffen (Hormone, Enzyme, Oberflächenantigene, usw.). Im weiteren soll am Beispiel einer leucinbedürftigen oder durch die bekannten Mutationsverfahren leucinbedürftig gemachten *Saccharomyces cerevisiae* und des Einsatzes von α-Ketoisocaproat, der Vorstufe bei der Biosynthese von Leucin in Bakterien, die weitere Durchführung besprochen werden. Um die Vorstufe wieder aus dem Medium entfernen zu können, kann das Enzym α-Ketoisovalerat-Dehydrogenase, das auch α-Ketoisovalerat (Biosynthesevorstufe für Valin in Bakterien) und α-Keto-β-methylvalerat (Biosynthesevorstufe für Isoleucin in Bakterien) umsetzt, als Zusatz des Mediums in freier oder gebundener Form an einem Träger zur Verwendung kommen. Die jeweils beste Darreichungsform des Enzyms muß durch Vorversuche ermittelt werden. Dieses Enzym benötigt außerdem als weitere Substrate NAD⁺ und Coenzym A im Medium Da die üblicherweise eingesetzten Medien Leucin enthalten, muß das Medium zunächst von Leucin befreit werden mit einem der bekannten Verfahren (siehe Beispiel 2). Ein Fusionsprodukt des kompletten Gens für die bakterielle Leucin-Aminotransferase, die α-Ketoisocaproat in Leucin überführt, und des Gens für die Invertase aus *Aspergillus oryzae* mit einem durchgängigen Leseraster ist eine geeignete Form für die direkte Transformation der Mitglieder der Population und wird mit den bekannten molekularbiologischen Verfahren hergestellt. Leucin-Aminotransferase benötigt als Substrate zusätzlich noch Glutamat und Pyridoxal-5-phosphat, die Sproßhefen in den meisten Fällen schon intrazellulär in ausreichenden Mengen besitzen.

### (1. Selektion und Anreicherung der benötigten molekularbiologisch modifizierten Mitglieder der Population durch das erfindungsgemäße Verfahren)

Die Selektion und Anreicherung der benötigten molekularbiologisch modifizierten Mitglieder der Population für das erfindungsgemäße Verfahren ist analog dem Beispiel 2. Zuerst wird das hergestellte Fusionsprodukt der Gene mit der direkten Transformation in die Mitglieder der Population überführt. Unter den Transformanten werden auch solche sein, die das Fusionsprodukt an Positionen in ihre genomische DNA integriert haben, an denen Gene des Zellzyklus liegen. Einige dieser Transformanten werden zum Beispiel ein funktionelles Genprodukt aus dem Zellzyklusgen, der Leucin-Aminotransferase und die Invertase exprimieren. Die Generationszeit der ursprünglich verwendeten *Saccharomyces cerevisiae* im jeweils üblicherweise eingesetzten Medium, den Kulturbedingungen und den verwendeten Kulturgefäßen muß als Richtwert für die weitere Vorgehensweise bekannt sein oder ermittelt werden. Die Pulsdauer bei verschiedenen zugesetzten α-Ketoisocaproat-Konzentrationen hängt von der dem Medium zugesetzten Menge an α-Ketoisovalerat-Dehydrogenase, dem verwendeten Medium mit den beigefügten Substanzen (NAD⁺, Coenzym A, evtl. Glutamat, evtl. Pyridoxal-5-phosphat), den verwendeten Kulturgefäßen und den Kulturbedingungen ab, und wird deshalb empirisch eingestellt. Eine Reihe von Medien mit dem zugesetztem Enzym mit verschiedenen Pulsdauern bei Zugabe einer bestimmten α-Ketoisocaproat-Konzentration wird angesetzt, deren Bereich über beispielsweise 1/20 der ermittelten Generationszeit und unterhalb der ermittelten Generationszeit liegt. Da der Bedarf der unterschiedlichen Transformanten im Gemisch an der essentiellen Aminosäure differiert, kann die jeweilige minimale Pulsdauer nicht bestimmt werden. Die 1/20 der ermittelten Generationszeit als kleinste Pulsdauer stellen deshalb nur ein Richtwert dar. Kürze Pulsdauern könnten selbstverständlich auch durchgeführt werden.

Jedes der angesetzten Medien wird nun in die Schalen der Multischalen in ausreichender Menge und mit ausreichendem Volumen pipettiert. Um Verwechslungen zu vermeiden, empfiehlt es sich, alle Schalen einer Multischale nur mit einem Medium zu füllen. Da bei allen Medien der Reihe die Vorgehensweise identisch ist, wird an einem Beispiel die weitere Durchführung besprochen. In die erste Schale einer Multischale mit 48 Schalen mit dem Medium, bei dem die Pulsdauer 1/15 der Generationszeit beträgt, wird ein Teil der erzeugten Transformanten pipettiert und anschließend der erste α-Ketoisocaproat-Puls verabreicht. Nach der Dauer einer Generationszeit wird der zweite α-Ketoisocaproat-Puls und periodisch in Zeitabständen der Dauer einer Generationszeit werden weitere α-Ketoisocaproat-Pulse verabreicht. Sobald eine gewisse Zelldichte erreicht ist, das M Medium verbraucht ist, das zugesetzte Enzym in seiner Aktivität sich vermindert oder sich unerwünschte Substanzen im Medium zu stark angehäuft haben, wird ein Aliquot der Zellen (z.B.: 10% bis 30%) der ersten Schale entnommen und in die zweite Schale überführt. In der zweiten Schale wird dann die periodische Verabreichung der α-Ketoisocaproat-Pulse fortgesetzt, bis wiederum eine gewisse Zelldichte erreicht ist, das Medium verbraucht ist oder unerwünschte Substanzen sich im Medium zu stark angehäuft haben. In diesem Fall wird ein Aliquot der Zellen der zweiten Schale entnommen und in die dritte Schale überführt. Die periodischen Verabreichung der α-Ketoisocaproat-Pulse und das Überführen jeweils eines Aliquots von Zellen in frisches Medium aus den oben genannten Gründen wird solange fortgesetzt, bis sich entweder bei dieser Kultur oder bei mindestens einer der Kulturen mit einer anderen α-Ketoisocaproat-Pulsdauer eine synchronisierte Population von Zellen nachweisen läßt. Falls aus den in Beispiel 1 genannten Gründen bei keiner Kultur eine Synchronisation nachweisbar ist, führt man das erfindungsgemäße Verfahren solange fort bis eine genügende Generationsanzahl (siehe Beispiel 1) von der Kultur durchlaufen wurde. Es versteht sich, daß bei der Ausführung des erfindungsgemäßen Verfahrens die optimalen Kulturbedingungen eingehalten werden oder nur eine sehr kurze Abweichung von den optimalen Kulturbedingungen verursacht wird. Gegebenenfalls kann man auch periodische Verabreichungen der α-Ketoisocaproat-Pulse in Vielfachen oder Kombinationen von Vielfachen der bestimmten Generationszeit einsetzen und/oder die Abstände der α-Ketoisocaproat-Pulse zueinander etwas größer als die bestimmte Generationszeit machen, um besser geeignete Transformanten zu erlangen. Die Kulturen mit den kürzesten α-Ketoisocaproat-Pulsdauern und den geringsten α-Ketoisocaproat-Konzentrationen werden aufgrund der in Beispiel 1 genannten Gründe zuerst nach geeigneten einzelnen Transformanten gescreent. Die Transformanten der einzelnen Kulturen werden mit den bekannten Verfahren separiert und vermehrt. Zum Screening nach denjenigen Transformanten, die zum Beispiel ein Fusionsprodukt des übertragenen Genkonstrukts mit einem zellzyklusabhängigen Gen besitzen, kann die Polymerasekettenreaktion oder die Hybridisierung mit spezifischen Sonden eingesetzt werden. Alternativ kann mit Durchflußzytometrie-Verfahren nach Transformanten gesucht werden, die eine zellzyklusabhängige Expression der übertragenen Gene zeigen (z.B.: Die Expression eines Zellzyklusgens und die Invertase-Aktivität zeigen eine Korrelation zueinander).

### (2. Anpassung des erfindungsgemäßen Verfahren auf Labor- und Technikmaßstab)

Um das erfindungsgemäße Verfahren bei *Saccharomyces cerevisiae* auch im Labor- und Technikmaßstab durchzuführen zu können, muß die pulsartige Verabreichung der Vorstufe im eingesetzten Medium auf die größeren Kulturgefäßen und Kulturvolumina angepaßt werden, da es durch die veränderte Geometrie sein kann, daß die bisherige Pulsdauer dazu nicht mehr ausreicht, daß die Mitglieder der synchronisierten Population in ausreichender Menge Leucin für sich bilden können. Durch die vorher durchgeführte Selektion, Anreicherung und der anschließenden Isolation von geeigneten molekularbiologisch modifizierten Mitgliedern der Population sind mindestens die Pulsdauer, die α-Ketoisocaproat-Konzentration, die Abstände zwischen den α-Ketoisocaproat-Pulsen für ein Kulturvolumen bekannt, welche als Richtwerte für das weitere Vorgehen dienen. Das Doppelte dieses Volumens an Medium mit der bekannten α-Ketoisovalerat-Dehydrogenase-Konzentration wird in einem Kulturgefäß vorgelegt, die Zellen der aufgrund des Screeningverfahrens als für geeignet befundenen Transformante zugegeben und anschließend der erste α-Ketoisocaproat-Puls verabreicht. Nach der bekannten Zeitdauer wird der zweite α-Ketoisocaproat-Puls und periodisch in den bekannten Zeitabständen weitere α-Ketoisocaproat-Pulse verabreicht. Sobald eine gewisse Zelldichte im Medium erreicht ist, das Medium verbraucht ist oder sich unerwünschte Substanzen im Medium zu stark angehäuft haben, wird ein Aliquot der Zellen (z.B.: 5% bis 10%) dem Kulturgefäß entnommen und in ein anderes Kulturgefäß mit frischem Medium überführt, wobei die Übertragung zwischen zwei α-Ketoisocaproat-Pulsen erfolgen sollte. Wenn feststeht, daß die Zellen sich bei diesem größeren Kulturvolumen wie gewohnt teilen, kann das Kulturvolumen um eine weitere Stufe (zum Beispiel auf das Doppelte) vergrößert werden. Falls die Zellen sich nicht wie gewohnt teilen, reicht eventuell die bisherige Zeitdauer der verabreichten α-Ketoisocaproat-Pulsen nicht mehr aus, damit die Zellen der synchronisierten Population in ausreichender Menge Leucin für sich bilden können. In diesem Fall wird die Pulsdauer, die α-Ketoisocaproat-Konzentration und der Zeitabstand zwischen den α-Ketoisocaproat-Pulsen soweit vorsichtig verändert, bis die Zellen wie gewohnt wachsen. Wenn das nächst größere Kulturvolumen nicht mehr geeignet ist für das verwendete Kulturgefäß, wird die Synchronisation in dem nächst größeren Kulturgefäß weiter durchgeführt. Wenn feststeht, daß die Zellen sich in diesem größeren Kulturgefäß wie gewohnt teilen, kann auch das Kulturvolumen vergrößert werden. Ansonsten ist wie oben beschrieben zu verfahren. Die obige Vorgehensweise zur Anpassung ist solange fortzusetzen, bis die synchronisierte Kultur ins gewünschte Kulturgefäß und ins gewünschte Kulturvolumen überführt worden ist. Bei großen Kulturvolumen muß man gegebenenfalls die Art der Erzeugung des α-Ketoisocaproat-Pulses auf eine andere Art der Erzeugung umstellen, wenn die bisherige Erzeugung ab einem gewissen Kulturvolumen Probleme bereitet.

Zur Gewinnung der Invertase werden die Zellen der synchronisierten Kultur beim Erreichen der auf Zellzahl normierten maximalsten Expressionsstärke sehr schnell mit einem der bekannten Verfahren abgetötet und die Zellen anschließend lysiert, wobei die endogenen Proteinasen/Proteasen durch entsprechende Chemikalienzugaben inaktiviert werden. Durch affinitätschromatographische Methoden wird aus dem Lysat der Zellen zunächst das Fusionsprodukt gebunden. Dabei kann die spezifische Gruppe, die an der Matrix des Gels gebundenen ist, ein spezifischer Antikörper gegen das Zellzyklusgenprodukt oder gegen die Leucin-Aminotransferase sein. Alternativ kann auch eines der Substrate der Leucin-Aminotransferase als spezifische Gruppe eingesetzt werden. Die optimalste spezifische Gruppe für die Isolation muß durch Vorversuche ermittelt werden. Die unerwünschten anderen Substanzen des Lysates werden nach Bindung des Fusionsproduktes durch Waschschritte vom Gel entfernt. Je nach eingesetzter Gruppe kann entweder die Invertase durch entsprechende proteolytische Spaltung in einem Batchverfahren vom gebundenen Fusionsprodukt freigesetzt oder das Fusionsprodukt durch Zugabe der gewählten Substrates der Leucin-Aminotransferase und/oder entsprechenden Pufferbedingungen eluiert werden. Im ersten Fall wird die Invertase weiteren Reinigungsschritten unterzogen. Im zweiten Fall wird die Invertase durch proteolytische Spaltung freigesetzt und anschließend weiter aufgereinigt.

## Patentansprüche

1. Verfahren zur Synchronisation von Zellteilungen einer Kultur oder Mischkultur mit jeweils mindestens zwei Mitgliedern mindestens einer Population,
dadurch gekennzeichnet, daß
a) die Mitglieder mindestens einer Population in der Kultur oder in der Mischkultur mindestens ein an mindestens ein zellzyklusabhängiges Gen oder Genprodukt direkt und/oder indirekt über Expressionsmechanismen und/oder andere zelluläre Regulationsmechanismen gekoppeltes eingebrachtes oder verändertes Gen oder Genprodukt besitzen, das durch diese Kopplung zellzyklusabhängig exprimiert oder aktiviert wird, und daß
b) mindestens eine Substanz, die entweder eine Vorstufe für mindestens eine mindestens wachstumsfördernde Substanz für die besagten Mitglieder der Population von a) ist, die durch das besagte gekoppelte Gen oder Genprodukt von a) direkt oder indirekt in die wachstumsfördernde Substanz umgesetzt wird, oder eine mindestens wachstumshemmende Substanz für die besagten Mitglieder der Population von a) ist, die durch das besagte gekoppelte Gen oder Genprodukt von a) direkt oder indirekt inaktiviert wird, oder eine Vorstufe für eine erst innerhalb der besagten Mitglieder der Population von a) zu einer mindestens wachstumshemmenden werdenden Substanz für die besagten Mitglieder der Population von a) ist, die und/oder dessen Folgeprodukt durch das besagte gekoppelte Gen oder Genprodukt von a) direkt oder indirekt inaktiviert wird, mindestens zweimal pulsartig und in zeitlichen Abständen gleich oder größer als die minimale Zeitdauer eines kompletten Zellzyklus/Teilungszyklus der besagten Mitglieder der Population von a) im jeweilig benutzten Kulturmedium verabreicht wird, so daß nur diejenigen besagten Mitglieder der Population von a) die zugeführte Substanz umsetzen/inaktivieren können, die innerhalb der Verabreichungszeit und in den Verabreichungsabständen das entsprechende gekoppelte Gen exprimieren oder über das entsprechende aktive Genprodukt verfügen.

2. Substanzverabreichung beim Verfahren nach Patentanspruch 1,
dadurch gekennzeichnet, daß
a) mindestens ein weiteres zusätzlich eingebrachtes oder verändertes zellzyklusgekoppeltes Gen oder Genprodukt der besagten Mitglieder der Population nach Patentanspruch 1 direkt oder indirekt mindestens ein Photon und/oder mindestens eine meßbare Substanz zellzyklusabhängig erzeugt, und daß
b) die pulsartige Zugabe der besagten Substanz nach Patentanspruch 1 für die besagten Mitglieder der Population von Patentanspruch 1 auf das Auftreten des Photons von a) und/oder auf das Auftreten der meßbaren Substanz von a) abgestimmt wird.

3. Herstellung oder Umsetzung mindestens einer Substanz mit mindestens einer nach dem Verfahren nach Patentanspruch 1 oder nach Patentanspruch 2 synchronisierten Kultur oder Mischkultur mit jeweils mindestens zwei Mitgliedern mindestens einer Population,
dadurch gekennzeichnet, daß
die besagten Mitglieder der Population mindestens ein weiteres zusätzlich eingebrachtes oder verändertes zellzyklusgekoppeltes Gen oder Genprodukt besitzen, das mindestens eine Substanz in einem bestimmten Zellzyklusabschnitt herstellt oder umsetzt.

4. Verfahren nach Patentanspruch 1,
dadurch gekennzeichnet, daß
a) die verabreichte mindestens wachstumshemmende Substanz oder die verabreichte Vorstufe für eine mindestens wachstumshemmende Substanz für die besagten Mitglieder von Patentanspruch 1 ein Antibotikum, Herbizid, Insektizid, Fungizid, Pestizid oder Nematozid ist, und daß
b) das an mindestens ein zellzyklusabhängiges Gen oder Genprodukt direkt und/oder indirekt über Expressionsmechanismen und/oder andere zelluläre Regulationsmechanismen gekoppelte eingebrachte oder veränderte Gen oder Genprodukt ein Resistenzgen/Resistenzgenprodukt ist, das durch Spaltung oder chemische Modifikation die besagte Substanz von a), besagte Vorstufe von a) oder die aus der besagten Vorstufe von a) mindestens wachstumshemmend gewordene Substanz inaktiviert.

5. Verfahren nach Patentanspruch 1,
dadurch gekennzeichnet, daß
a) die verabreichte Vorstufe für eine mindestens wachstumsfördende Substanz für die besagten Mitglieder nach Patentanspruch 1 eine Vorstufe für eine essentielle Aminosäure oder für ein Vitamin ist, und daß
b) das an mindestens ein zellzyklusabhängiges Gen oder Genprodukt direkt und/oder indirekt über Expressionsmechanismen und/oder andere zelluläre Regulationsmechanismen gekoppelte eingebrachte oder veränderte Gen oder Genprodukt die besagte Vorstufe von a) in die besagte essentielle Aminosäure von a) oder das besagte Vitamin von a) überführt.

6. Verfahren nach Patentanspruch 1,
dadurch gekennzeichnet, daß
das zellzyklusabhängige Gen oder Genprodukt ein G1- oder G2-Cyclin ist, an das über direkte und/oder indirekte Expressionsmechanismen und/oder andere zelluläre Regulationsmechanismen das besagte eingebrachte oder veränderte Gen oder Genprodukt nach Patentanspruch 1 gekoppelt ist.

7. Verfahren nach Patentanspruch 1,
dadurch gekennzeichnet, daß
der Substanz-Puls erzeugt wird durch Austausch von mindestens einem Medium mit der verabreichten Substanz und mindestens einem Medium ohne die verabreichte Substanz, durch Umsatz/Abbau der verabreichten Substanz durch mindestens ein Enzym und/oder durch Entfernung der verabreichten Substanz mittels Bindung an mindestens einen spezifischen Liganden für die Substanz.
